# EUROPÄISCHE PATENTSCHRIFT

(11) **EP 1 570 266 B1**
(45) Veröffentlichungstag und Bekanntmachung des Hinweises auf die Patenterteilung: **28.02.2007**
(21) Anmeldenummer: 03789105.8
(22) Anmeldetag: 04.12.2003
(51) Int. Cl.: G01N 33/50

(54) **VERFAHREN ZUR MESSUNG DER AKTIVITÄT G(ALPHA)I- ODER G(ALPHA)O-GEKOPPELTER REZEPTOREN MITTELS CA2+-INFLUX IN ZELLEN**
METHODS FOR MEASURING THE ACTIVITY OF G(ALPHA)I-COUPLED OR G(ALPHA)O-COUPLED RECEPTORS BY MEANS OF THE CA2+ INFLUX IN CELLS
PROCEDE POUR MESURER L'ACTIVITE DE RECEPTEURS COUPLES A G(ALPHA)I OU G(ALPHA)O AU MOYEN DE L'INFLUX DE CA2+ DANS DES CELLULES

(30) Priorität: 05.12.2002 DE 10256947
(43) Veröffentlichungstag der Anmeldung: 07.09.2005
(73) Patentinhaber: Grünenthal GmbH, 52078 Aachen (DE)
(72) Erfinder: WADE, Erik James, 79576 Weil am Rhein (DE); JANOCHA, Elke, 52441 Linnich (DE); GERMANN, Tieno, 52080 Aachen (DE); BAHRENBERG, Gregor, 52074 Aachen (DE)
(74) Vertreter: Hiebl, Inge Elisabeth
(86) Internationale Anmeldenummer: PCT/EP2003/013510
(87) Internationale Veröffentlichungsnummer: WO 2004/051264

(56) Entgegenhaltungen:
- WO-A-00/49416
- US-A1- 2002 051 980
- GOPALAKRISHNAN S M ET AL: "A cell-based microarrayed compound screening format for identifying agonists of G-protein-coupled receptors" ANALYTICAL BIOCHEMISTRY, ACADEMIC PRESS, SAN DIEGO, CA, US, Bd. 321, Nr. 2, 15. Oktober 2003 (2003-10-15), Seiten 192-201, XP004457157 ISSN: 0003-2697
- STABLES JENNY ET AL: "A bioluminescent assay for agonist activity at potentially any G-protein-coupled receptor" ANALYTICAL BIOCHEMISTRY, Bd. 252, Nr. 1, 1997, Seiten 115-126, XP002274702 ISSN: 0003-2697
- COWARD PETER ET AL: "Chimeric G proteins allow a high-throughput signaling assay of Gi-coupled receptors" ANALYTICAL BIOCHEMISTRY, Bd. 270, Nr. 2, 1. Juni 1999 (1999-06-01), Seiten 242-248, XP002274703 ISSN: 0003-2697

## Beschreibung

Die vorliegende Erfindung betrifft Verfahren zur Messung der Aktivierung oder Deaktivierung G(alpha)i bzw. G(alpha)o-gekoppelter Rezeptoren sowie Verfahren zur Identifizierung von Agonisten oder Antagonisten derartiger Rezeptoren.

G-Protein-gekoppelte Rezeptoren (engl. "G protein coupled receptors", GPCR) sind eine umfangreiche Familie von Proteinen, die bei der Signaltransduktion in Zellen eine wesentliche Rolle spielen. Die Bezeichnung "GPCR" leitet sich von ihrer Assoziierung mit einem heterotrimeren Komplex aus G(alpha), G(beta) und G(gamma)-Untereinheiten ab. Die G(alpha)-Untereinheiten der bei der synaptischen Transmission von Signalen beteiligten Rezeptoren können grob aufgrund ihrer Funktion und Kopplung mit den GPCR eingeteilt werden. Die Mitglieder der G(alpha)s-Familie stimulieren die Aktivität von Adenylatcyclasen, während diejenigen der G(alpha)i/o-Familie die Aktivität von Adenylatcyclasen hemmt. Die Proteine der G(alpha)q- und G(alpha)12/13-Familie sind wirksame Stimulatoren der Aktivität der Phospholipase C(beta). Diese Subtypen zeigen jedoch keine Wirkung bezüglich der Adenylatcyclase-Aktivität. Üblicherweise interagiert ein gegebener GPCR nur mit einer einzigen Familie der G(alpha)-Proteine, obwohl einige Ausnahmen von dieser Regel bekannt sind.

Der Name der G(alpha)-Proteine leitet sich von ihrer Fähigkeit zur Bindung von Guanosindi- oder -triphosphat (GDP oder GTP) ab, welches als Schalter fungiert, der die Aktivität und die Assoziierung des G(alpha)-Proteins mit dem GPCR und den G(beta)/G(gamma)-Untereinheiten reguliert. GDP bindet an G(alpha)-Proteine in ihrem inaktiven Zustand, in welchem sie nicht-kovalent mit den G(beta)/G(gamma)-Untereinheiten und einem entsprechenden GPCR assoziiert vorliegen. Die GPCR-Aktivierung führt zu allosterischen Konfirmationsänderungen des Rezeptors, was zur Dissoziation des G-Protein-Heterotrimers vom Rezeptor und zur Dissoziation des gebundenen GDP von der G(alpha)-Komponente führt. Die intrazellulären Konzentrationen von GTP übersteigen die GDP-Konzentrationen üblicherweise um mehrere Größenordnungen. Daher führt die Dissoziation von GDP von der G(alpha)-Untereinheit zur Bindung von GTP. Die Bindung von GTP an die G(alpha)-Untereinheit ergibt eine allosterische Konfirmationsumwandlung, die zur Dissoziation der G(alpha)-Untereinheit von den beta- und gamma-Untereinheiten sowie zur Aktivierung der Effektorfunktionen der alpha-Untereinheit führt. Die beta- und gamma-Bestandteile bleiben fest miteinander verbunden und bilden daher eine einzige funktionelle Einheit. Sobald sie aus dem Komplex mit der G(alpha)-Untereinheit und vom GPCR freigesetzt werden, führen sie verschiedene Effektorfunktionen unabhängig vom G(alpha)-Bestandteil aus. Der dargelegte G-Protein-Zyklus wird durch die Hydrolyse des an die G(alpha)-Untereinheit gebundenen GTP durch deren intrinsische GTPase-Aktivität geschlossen. Durch diesen Schritt kehrt die G(alpha)-Untereinheit wieder in den ursprünglichen Zustand zurück, was zur Reassoziation mit den beta/gamma-Untereinheiten und schließlich wieder zur Bindung des Heterotrimers an den GPCR führt.

Die Messung der Aktivität G(alpha)q-gekoppelter Rezeptoren aufgrund der Messung der cytoplasmatischen Ca²⁺-Konzentration, bspw. in lebenden Zellen mit kleiner, Zellmembran-permeabler Moleküle, wie Fluoreszenzfarbstoffen, die ihre Fluoreszenzeigenschaften nach Bindung von Ca²⁺ verändern, ist im Stand der Technik bekannt. Diese Verfahren beruhen darauf, daß G(alpha)q-Proteine die Phospholipase C(beta) aktivieren, welche die Spaltung von Phosphatidylinositol-(4,5)-bisphosphat (PIP2) zu Inositoltriphosphat (IP3) und Diacylglycerol (DAG) katalysiert. Im Gegensatz zu PIP2, das ein integrales Membramlipid ist, liegt IP3 im Cytosol gelöst vor. Somit kann durch die Wirkung der Phospholypase C(beta) freigesetztes IP3 zu IP3-Rezeptor-Calciumkanälen des endoplasmatischen Ritikulums (ER) difundieren und die Freisetzung von Ca²⁺ aus dem ER bewirken. Die dadurch erhöhte cytoplasmatische Ca²⁺-Konzentration korreliert direkt mit der Aktivierung des GPCR, weshalb die Messung des cytoplasmatischen Ca²⁺ die indirekte Messung der Rezeptoraktivierung erlaubt. Mit derartigen Verfahren können bspw. potentielle Liganden des betrachteten Rezeptors hinsichtlich ihrer agonistischen oder antagonistischen Eigenschaften bewertet werden.

Die Messung der Aktivität G(alpha)i- bzw. G(alpha)o-gekoppelter Rezeptoren erweist sich im Gegensatz dazu als sehr viel schwieriger. Wie vorstehend dargelegt, wirken G(alpha)i- bzw. G(alpha)o-Untereinheiten auf die Adenylatcyclase. Ein möglicher Ansatz besteht daher in der Messung des Produkts dieses Enzyms, dem zyklischen 3'-5'-Adenosinmonophosphat (cAMP). Die Messung von cAMP ist jedoch teuer, zeitraubend und wird durch einen relativ kleinen dynamischen Bereich des Tests begrenzt. Bei einem weiteren Verfahren wird ein chimäres G(alpha)-Protein in die Zellen eingebracht, bei dem die Wechselwirkung mit dem in Rede stehenden G(alpha)i- bzw. G(alpha)o-gekoppelten GPCR erhalten bleibt, während die stromabwärtige Effektorwirkung des G(alpha)-Proteins von der Inhibition der Adenylatcyclase zur Aktivierung der Phospolypase C(beta) geändert wird, so daß die Bestimmung der GPCR-Aktivität wieder durch Messung des cytoplasmatischen Ca²⁺ ermöglicht wird (vgl. Coward et al. (1999) Anal. Biochem. 270(2): 242-248). Diese Technik erfordert jedoch einen zusätzlichen, zeitaufwendigen Klonierungsschritt zur Bereitstellung der G(alpha)-Chimäre, insbesondere, falls stabile Transfektanden benötigt werden. Des weiteren können aufgrund der artifiziellen Natur der Chimäre von der tatsächlichen *in vivo* Situation abweichende, artifizielle Ergebnisse nicht ausgeschlossen werden.

Bei einem weiteren Verfahren wird ein promiskuitives G(alpha)-Protein in die Zellen eingebracht, das sowohl mit G(alpha)i- bzw. G(alpha)o-gekoppelten GPCR als auch mit G(alpha)q-gekoppelten GPCR wechselwirken kann, so dass die Bestimmung der GPCR-Aktivität wieder durch die Messung des cytoplasmatischen Ca²⁺ ermöglicht wird (vgl. Stables et al. (1997) Anal. Biochem. 252(1): 115-126).

Der vorliegenden Erfindung liegt daher die Aufgabe zugrunde, ein neues Verfahren zur Messung der Aktivität G(alpha)i- bzw. G(alpha)o-gekoppelter Rezeptoren bereitzustellen, das die Nachteile von im Stand der Technik bekannten Verfahren überwindet.

Diese Aufgabe wird durch die in den Ansprüchen gekennzeichneten Ausführungsformen der vorliegenden Erfindung gelöst.

lnsbesondere wird ein Verfahren zur Messung der Aktivierung eines G(alpha)i- und/oder G(alpha)o-gekoppelten Rezeptors bei Zellen, die mindestens einen G(alpha)q-gekoppelten Rezeptor exprimieren, umfassend:
(a) Gleichzeitiges Behandeln der Zellen mit einer derartigen Menge (bzw. einer derartigen Konzentration) eines Agonisten des (mindestens einen, d.h. im Falle mehrerer irgendeines der mehreren) G(alpha)q-gekoppelten Rezeptors, daß sich eine untergrenzwetige Aktivität ergibt, und mit einem Agonisten des G(alpha)i- und/oder G(alpha)o-gekoppelten Rezeptors, und
(b) Messen der cytoplasmatischen Ca²⁺-Konzentration der Zellen,
bereitgestellt.

Das erfindungsgemäße Meßverfahren erlaubt eine indirekte Messung der Aktivität von G(alpha)i/o-gekoppelter GPCR aufgrund einer synergistischen Wechselwirkung zwischen aktivierten G(alpha)q-gekoppelten GPCR und G(alpha)i/o- gekoppelten GPCR. Insbesondere wird Phospholipase C(beta) (im folgenden mit PLC(beta)) abgekürzt) in zwei getrennten Phasen aktiviert. Während der ersten Phase bringen freie G(beta)/G(gamma)-Untereinheiten PLC(beta) zur Plasmamembran, wodurch das Enzym und ihr Substrat (PIP2) nahe zusammengebracht werden. Während der zweiten Phase aktivieren G(alpha)q-Untereinheiten die enzymatische Aktivität von PLC(beta). Unter Verwendung eines Agonisten des G(alpha)q-gekoppelten Rezeptors, bspw. ATP oder UTP, zur Auslösung einer bestimmten, untergrenzwertigen, Aktivierung von G(alpha)q-Rezeptoren, kann daher die Aktivität G(alpha)i/o-gekoppelter Rezeptoren in einfacher Weise wirksam gemessen werden.

Erfindungsgemäß ist die Menge oder Konzentration des Agonisten des G(alpha)q-gekoppelten Rezeptors, die eine "untergrenzwertige" Aktivität ergibt, insbesondere diejenige, bei welcher das Verhältnis des Ergebnisses einer Messung der cytoplasmatischen Ca²⁺-Konzentration von Zellen, die mit einer bestimmten Menge eines Agonisten des G(alpha)q-gekoppelten Rezeptors behandelt wurden, zum Ergebnis der Messung der cytoplasmatischen Ca²⁺-Konzentration von Zellen, welche mit der gleichen Menge (bzw. Konzentration) des vorstehend genannten Agonisten des G(alpha)q-gekoppelten Rezeptors und gleichzeitig mit einer Menge (Konzentration) eines Agonisten eines G(alpha)i- und/oder G(alpha)o-gekoppelten Rezeptors, welche zu dessen vollständiger Aktivierung ausreicht, behandelt werden, möglichst klein, d.h. nicht größer als etwa 1:3 ist. Vorzugsweise ist dieses Verhältnis nicht größer als etwa 1:10, insbesondere nicht größer als etwa 1:20, besonders bevorzugt ist es für das jeweilige Paar von Agonisten der Rezeptoren minimal. Selbstverständlich kann das obige Verhältnis der Messergebnisse auch umgekehrt gebildet werden (also cytoplasmatische Ca²⁺-Konzentration nach gleichzeitiger Behandlung einem Agonisten eines G(alpha)q-Rezeptors und einem Agonisten des G(alpha)i/o-gekoppelten Rezeptors zu cytoplasmatischer Ca²⁺-Konzentration nach Behandlung nur mit einem Agonisten eines G(alpha)q-gekoppelten Rezeptors). In diesem Fall entspricht die eine untergrenzwertige Aktivität des G(alpha)q-gekoppelten Rezeptors auslösende Menge oder Konzentration des Agonisten derjenigen Menge bzw. Konzentration, bei der das Verhältnis der Messungen der cytoplasmatischen Ca²⁺-Konzentrationen möglichst groß, d.h. nicht kleiner als etwa 3:1, vorzugsweise mindestens etwa 10:1, insbesondere zumindest etwa 20:1 ist.

Das bedeutet, daß die Menge (Konzentration) des Agonisten des G(alpha)q-gekoppelten Rezeptors, die eine untergrenzwertige Aktivität ergibt, diejenige ist, bei welcher eine möglichst große Signalverstärkung bei einer Messung der cytoplasmatischen Ca²⁺-Konzentration festgestellt wird, wenn die Zellen gleichzeitig mit dem Agonisten des G(alpha)q-gekoppelten Rezeptors und mit dem Agonisten des in Rede stehenden G(alpha)i/o-gekoppelten Rezeptors behandelt werden, im Vergleich zu einer Messung der Ca²⁺-Konzentration, die sich bei einer Behandlung der Zellen nur mit der gleichen Menge oder Konzentration des Agonisten des G(alpha)q-gekoppelten Rezeptors ergibt. Da die cytoplasmatische Ca²⁺-Konzentration ein Maß für die Aktivität der Phospholipase C(beta) ist, kann selbstverständlich das erfindungsgemäße Verfahren und die Bestimmung der Menge des Agonisten des G(alpha)q-gekoppelten Rezeptors, die eine untergrenzwertige Aktiviät ergibt, mit Hilfe jedes anderen geeigneten Tests zur Bestimmung der Aktivität der Phospholipase C(beta) durchgeführt werden.

Das erfindungsgemäße Messverfahren eignet sich auch insbesondere zur Identifzierung von Agonisten eines gegebenen G(alpha)i- bzw. G(alpha)o-gekoppelten Rezeptors. Einen weiteren Gegenstand der vorliegenden Erfindung bildet daher ein derartiges ldentifizierungsverfahren, das (i) das Bereitstellen von Zellen, die den G(alpha)i- und/oder G(alpha)o-gekoppelten Rezeptor und mindestens einen G(alpha)q-gekoppelten Rezeptor exprimieren, und (ii) das Durchführen des obigen Messverfahrens gemäß den Schritten (a) und (b) umfasst. Dabei wird selbstverständlich im Gegensatz zum erfindungsgemäßen Messverfahren im Schritt (a) kein (bekannter) Agonist des G(alpha)i- bzw. G(alpha)o-gekoppelten Rezeptors verwendet, sondern es wird eine Testsubstanz eingesetzt, deren Wirkung auf den in Rede stehenden GPCR zu überprüfen ist.

Das Messprinzip der vorliegenden Erfindung kann nicht nur zur Messung der Aktivierung G(alpha)i- bzw. G(alpha)o-gekoppelter Rezeptoren durch entsprechende Agonisten eingesetzt werden, sondern ebenfalls zur Messung der Deaktivierung bzw. Verhinderung der Aktivierung aufgrund von Antagonisten dieser Rezeptoren. Daher stellt die vorliegende Erfindung ein Verfahren zur Messung der Deaktivierung eines G(alpha)i- und/oder G(alpha)o-gekoppelten Rezeptors bei Zellen, die mindestens einen G(alpha)q-gekoppelten Rezeptor exprimieren, bereit, umfassend die Schritte:
(A) Gleichzeitiges Behandeln der Zellen mit einer Menge (bzw. einer Konzentration) eines Agonisten des G(alpha)q-gekoppelten Rezeptors derart, daß sich eine untergrenzwertige Aktivität ergibt, und mit einer Menge (Konzentration) eines Agonisten des G(alpha)i- und/oder G(alpha)o-gekoppelten Rezeptors, die gerade zur vollständigen Aktivierung ausreicht;
(B) Messen der cytoplasmatischen Ca²⁺-Konzentration der Zellen;
(C) Gleichzeitiges Behandeln der Zellen mit der gleichen Menge (bzw. Konzentration) von Schritt (A) des Agonisten des G(alpha)q-gekoppelten Rezeptors, mit der gleichen Menge (bzw. Konzentration) von Schritt (A) des Agonisten des G(alpha)i- und/oder G(alpha)o-gekoppelten Rezeptors und mit einem Antagonisten des G(alpha)i- und/oder G(alpha)o-gekoppelten Rezeptors;
(D) Messen der cytoplasmatischen Ca²⁺-Konzentration der Zellen; und
(E) Vergleichen der Ergebnisse aus den Messungen der Schritte (B) und (D).

Die Menge (oder Konzentration) des Agonisten des G(alpha)q-gekoppelten Rezeptors, die eine untergrenzwertige Aktivität ergibt, ist dabei wie vorstehend hinsichtlich des erfindungsgemäßen Verfahrens zur Messung der Aktivierung eines G(alpha)i- und/oder G(alpha)o-gekoppelten Rezeptors definiert.

Erfindungsgemäß können die Zellen im Schritt (C) auch zunächst mit dem Antagonisten des G(alpha)i- und/oder G(alpha)o-gekoppelten Rezeptors behandelt werden und danach in einem getrennten Schritt gleichzeitig mit dem Agonisten des G(alpha)q-gekoppelten Rezeptors und dem Agonisten des G(alpha)i- und/oder G(alpha)o-gekoppelten Rezeptors.

Ergibt die Messung der cytoplasmatischen Ca²⁺-Konzentration im Schritt (D) einen kleineren Wert als im Schritt (B), so liegt eine Deaktivierung des in Rede stehenden Rezeptors durch den Antagonisten vor. Der Schritt (E) kann daher das Bilden der Differenz oder eines Quotienten der Messergebnisse aus den Schritten (B) und (D) umfassen. Durch entsprechende Auftragungen dieser Differenzen bzw. Quotienten in Abhängigkeit von der Menge bzw. Konzentration des Antagonisten können durch einem Fachmann bekannte Methoden für den jeweiligen Rezeptor bzw. den Antagonisten charakteristische Werte, bspw. Inhibitionskonstanten, IC50-Werte usw., bestimmt werden. Gleiches gilt selbstverständlich auch für den Agonisten, bei konzentrationsabhängigen Messreihen bezüglich des vorstehenden Verfahrens zur Messung des Aktivierung eines G(alpha)i- und/oder G(alpha)o-gekoppelten Rezeptors.

Auch das erfindungsgemäße Messverfahren zur Bestimmung der Deaktivierung eines G(alpha)i- und/oder G(alpha)o-gekoppelten Rezeptors aufgrund eines Antagonisten kann im Rahmen eines Verfahrens zur Identifizierung solcher Antagonisten vorteilhaft eingesetzt werden. Ein derartiges Identifizierungsverfahren umfasst daher (I) das Bereitstellen von Zellen, die den G(alpha)i- und/oder G(alpha)o-gekoppelten Rezeptor und mindestens einen G(alpha)q-gekoppelten Rezeptor exprimieren, und (II) das Durchführen der Schritte (A) bis (E) gemäß dem vorstehenden Messverfahren. Selbstverständlich wird dabei wiederum im Schritt (C) eine Testsubstanz, deren antagonistische Wirkung bezüglich des in Rede stehenden GPCRs zu testen ist, anstatt eines bekannten Antagonisten verwendet.

Die vorstehend definierte Menge bzw. Konzentration des Agonisten des G(alpha)i- und/oder G(alpha)o-gekoppelten Rezeptors, die eine untergrenzwertige Aktivität ergibt, wird gemäß vorteilhafter Ausführungsformen der erfindungsgemä-ßen Verfahren durch eine empirische Vorgehensweise ermittelt. Dazu werden folgende Schritte durchgeführt:
(1) Behandeln der Zellen mit unterschiedlichen Mengen (bzw. Konzentrationen) des Agonisten des G(alpha)q-gekoppelten Rezeptors;
(2) Messen der cytoplasmatischen Ca²⁺-Konzentration der Zellen für jede Menge (Konzentration) des Agonisten von Schritt (1);
(3) Gleichzeitiges Behandeln der Zellen mit unterschiedlichen Mengen (oder Konzentrationen) des Agonisten des G(alpha)q-gekoppelten Rezeptors und einer jeweils konstanten Menge (Konzentration) des Agonisten des G(alpha)i- und/oder G(alpha)o-gekoppelten Rezeptors, die zur vollständigen Aktivierung des Rezeptors ausreicht;
(4) Messen der cytoplasmatischen Ca²⁺-Konzentration der Zellen für jede Menge (Konzentration) des Agonisten des G(alpha)q-gekoppelten Rezeptors von Schritt (3); und
(5) Vergleichen der Ergebnisse, insbesondere Bilden des Verhältnisses der Messungen der Schritte (2) und (4) für jeweils gleiche Mengen (Konzentrationen) des Agonisten des G(alpha)q-gekoppelten Rezeptors.

Vorzugsweise wird mit Hilfe der obigen Vorgehensweise die Menge oder Konzentration des Agonisten des G(alpha)q-gekoppelten Rezeptors, die eine untergrenzwertige Aktivität ergibt, als diejenige festgelegt, bei welcher sich ein Verhältnis der Meßergebnisse der Schritte (2) und (4) ergibt, das nicht größer als etwa 1:3, vorzugsweise nicht größer als etwa 1:10, insbesondere nicht größer als etwa 1:20 ist. Selbstverständlich kann auch hier wiederum der Kehrwert als Bezugsgröße verwendet werden, wobei entsprechend ein Verhältnis von nicht weniger als etwa 3:1, vorzugsweise mindestens etwa 10:1, mehr bevorzugt mindestens etwa 20:1, eine die untergrenzwertige Aktivität des G(alpha)q-gekoppelten Rezeptors hervorrufende Menge (oder Konzentration) des Agonisten dieses Rezeptors anzeigt.

Neben der Messung der Aktivität/lnhibition von G(alpha)i/o-gekoppelten Rezeptoren aufgrund der vorstehend dargelegten synergistischen Wechselwirkung mit G(alpha)q-gekoppelten Rezeptoren sind darüber hinaus auch entsprechende Verfahren möglich, welche sich eine entsprechende synergistische Wechselwirkung zwischen G(alpha)q-gekoppelten und G(alpha)s-gekoppelten Rezeptoren, zwischen G(alpha)12/13-gekoppelten und G(alpha)i/o-gekoppelten Rezeptoren bzw. zwischen G(alpha)12/13-gekoppelten und G(alpha)s-Rezeptoren zu nutze machen. So sind entsprechende Verfahren zur Messung der Aktivität/Inhibition von G(alpha)s-gekoppelten Rezeptoren aufgrund einer synergistischen Wechselwirkung mit G(alpha)q-gekoppelten Rezeptoren möglich. Weiter sind auch entsprechende Verfahren zur Messung der Aktivität/Inhibition von G(alpha)i/o-gekoppelten Rezeptoren aufgrund einer synergistischen Wechselwirkung mit G(alpha)12/13-gekoppelten Rezeptoren möglich. Des Weiteren sind auch entsprechende Verfahren zur Messung der Aktivität/Inhibition von G(alpha)s-gekoppelten Rezeptoren unter Verwendung einer synergistischen Wechselwirkung mit G(alpha)12/13-gekoppelten Rezeptoren möglich. Bei den genannten Verfahren der vorliegenden Erfindung werden dabei die in den vorstehend definierten Verfahrensschritten angegebenen Rezeptoren und deren Agonisten bzw. Antagonisten durch die jeweils zu messenden Rezptoren und deren Agonisten/Antagonisten und die jeweils mit diesen zu messenden Rezeptoren synergistisch wechselwirkenden Rezeptoren und deren Agonisten ersetzt.

Die Art und Weise der Messung der cytoplasmatischen Ca²⁺-Konzentration der Zellen ist erfindungsgemäß in keiner Weise eingeschränkt. Entsprechende Meßverfahren sind im Stand der Technik bekannt. Bspw. sind zur Messung der cytoplasmatischen Ca ²⁺-Konzentration geeignete Kits im Handel erhältlich, z.B. der FLIPR^{®} Calcium Plus Assay Kit von Molecular Devices. Üblicherweise erfolgt die Messung der cytoplasmatischen Ca²⁺-Konzentration unter Verwendung Ca²⁺-abhängiger Farbstoffe. Dies bedeutet, daß der in Rede stehende Farbstoff aufgrund der Bindung von Ca²⁺-Ionen in irgendeiner Weise seine spektralen Eigenschaften verändert, was somit der photometrischen Detektion zugänglich ist. Die "Bindung" eines oder mehrerer Ca²⁺-lonen an den jeweiligen Farbstoff ist dabei nicht eingeschränkt, oft handelt es sich jedoch hierbei um eine Komplexierung eines oder mehrerer Calcium-lonen durch das Farbstoffmolekül.

Aufgrund besonderer Vorteile, insbesondere hinsichtlich Genauigkeit und Empfindlichkeit der Messung, sind Fluoreszenzfarbstoffe, insbesondere solche, die kleine, Zellmembran-permeabler Moleküle darstellen, für die Bestimmung der cytoplasmatischen Ca²⁺-Konzentration besonders bevorzugt. Diese Moleküle ändern daher bei der Bindung von Calcium-lonen ihre Fluoreszenzeigenschaften. Besonders geeignete Ca²⁺-abhängige Fluoreszenzfarbstoffe zur Verwendung in den erfindungsgemäßen Verfahren sind bspw. Fluoreszenzfarbstoffe der Molekülfamilien Fura (bspw. Fura-2, Fura-4F, Fura-5F, Fura-6F, Fura-FF, Fura Red, Mag-Fura, Bis-Fura, wie Bis-Fura-2, insbesondere Zellmembran-permeable Derivate dieser Verbindungen, wie deren Ester, z.B. die Acetoxymethylester), Indo (bspw. Indo-1, Indo-5F und Mag-lndo-1 sowie Zellmembran-permeable Derivate davon, insbesondere geeignete Ester wie die Acetoxymethylester dieser Verbindungen), Quin (z.B. Quin-2 und Quin-2-Ester, wie Quin-2-Acetoxymethylester), Coumarinbenzothiazole (bspw. BTC und deren Esterderivate, insbesondere BTC-Acetoxymethylester), Fluo (bspw. Fluo-3, Fluo-4, Fluo-5F, Fluo-4FF, Fluo-5N und Mag-fluo-4 sowie geeignete Ester dieser Verbindungen wie die Acetoxymethylesther), Calcium Green (z.B. Calcium Green 1, 2 und 5N sowie geeignete Ester dieser Verbindungen wie die Acetoxymethylester), Oregon Green (bspw. Oregon Green 488 BAPTA-1, -2, -6F und -5N sowie geeignete Ester dieser Verbindungen wie die Acetoxymethylester), Calcium Orange, Calcium Crimson, Magnesium Green und deren geeignete Ester, insbsondere die Acetoxymethylester, Rhod (z.B. Rhod 2, -FF und -5N sowie geeignete Ester dieser Verbindungen wie die Acetoxymethylester) und X-rhod (bspw. X-rhod-1, -5F und FF sowie geeignete Ester dieser Verbindungen wie die Acetoxymethylester), die sämtlich im Handel erhältlich sind (Molecular Probes).

In den erfindungsgemäßen Verfahren können die zu analysierenden Rezeptoren bzw. die Rezeptoren, bezüglich denen bei Testsubstanzen eine agonistische oder antagonistische Aktivität festgestellt werden soll, also der jeweilige G(alpha)i-bzw. G(alpha)o-gekoppelte Rezeptor, aber auch der jeweilige G(alpha)q-gekoppelte Rezeptor in den Zellen sowohl endogen als auch exogen exprimiert werden. Im letzteren Fall sind die Zellen daher mit einem für den oder die jeweiligen Rezeptor(en) codierenden Nukleinsäurekonstrukt, das für die Expression dieses oder dieser Rezeptors/Rezeptoren sorgt, transfiziert. Dabei können die Zellen entweder transient zur vorübergehenden Expression des jeweiligen Rezeptors transfiziert sein, oder es kann ein Konstrukt zur stabilen Integration in das Genom der jeweiligen Zellen verwendet werden, um für eine stabile Expression des Rezeptors zu sorgen. Selbstverständlich sind hinsichtlich der Expression der entsprechenden Rezeptoren erfindungsgemäß jegliche Kombinationen, je nach erwünschter Eigenschaft, der vorstehend dargelegten Expressionsmöglichkeiten geeignet. So können bspw. Zellen verwendet werden, die sowohl den G(alpha)q-gekoppelten als auch den jeweiligen G(alpha)i/o-gekoppelten Rezeptor endogen exprimieren. Es kann aber auch nur der G(alpha)q-gekoppelte oder nur der G(alpha)b/o-gekoppelte Rezeptor endogen von den Zellen exprimiert werden, während der andere Rezeptortyp von den Zellen aufgrund einer Transfektion (transient oder stabil) mit einem entsprechenden Nukleinsäurekonstrukt, insbesondere einem zur Expression geeigneten Vektor, exogen exprimiert wird. Selbstverständlich ist es auch möglich, beide Rezeptortypen mittels der entsprechenden Konstrukte heterolog in den Zellen zu exprimieren.

Durch die Möglichkeit der exogenen Expression von Rezeptoren, insbesondere des jeweiligen G(alpha)i/o-gekoppelten Rezeptors, können erfindungsgemäß auch gegenüber dem Wildtyp-Rezeptor veränderte Fragmente, Derivate, Allele und Mutanten exprimiert und so in den erfindungsgemäßen Verfahren eingesetzt werden. Bspw. ist es so möglich, die Aktivität eines G(alpha)i- bzw. G(alpha)o-gekoppelten Rezeptors, der aufgrund einer Derivatisierung oder einer Mutation eine veränderte Bindung gegenüber einem jeweiligen Agonisten oder Antagonisten aufweist, bezüglich seiner Aktivierung oder Deaktivierung durch den entsprechenden Agonisten bzw. Antagonisten zu untersuchen. Die entsprechende Veränderung kann selbstverständlich derart sein, daß sich eine stärkere oder schwächere Bindung des Liganden, d.h. des Agonisten oder Antagonisten, ergibt.

Daher ist der G(alpha)i- bzw. G(alpha)o-gekoppelte Rezeptor erfindungsgemäß keinerlei Einschränkungen unterworfen, Beispiele sind Opioid-Rezeptoren (bspw. µ, κ, δ und ORL1), P2Y12, Edg-Familie, GABA-B, Muscarin-M2, -M4, Dopamin-D2, -D3, -D4, Histamin-H3, Serotonin-5HT1- Familie, C3a-, C5a-, fMLP-, CXCR1-5-, CCR1-9-, XCR1-, CX3CR1-, Neuropeptid-Y1 bis 6-, Somatostatin-Sst2, "Chemoattractant R-homologous molecule expressed on Th2 cells", Prostaglandin-E-Typ 3-, Adenosin-A1- und Adenosin-A3-Rezeptoren.

Auch hinsichtlich des G(alpha)q-gekoppelten Rezeptors bestehen erfindungsgemäß keinerlei Einschränkungen. Es können daher sowohl Wildtyp-Rezeptoren als auch Fragmente, Allele, Derivate oder Mutanten von G(alpha)q-gekoppelten Rezeptoren verwendet werden, mit der Maßgabe, daß das jeweilige Fragment, Allel, Derivat bzw. die jeweilige Mutante einen Agonisten zu binden und die vorstehend dargelegte dynamische Wechselwirkung zwischen den Rezeptorsystemen auf Grundlage der jeweiligen G-Proteine auszulösen vermag. In einer keineswegs abschließenden Aufzählung können als Beispiele von erfindungsgemäß verwendbaren G(alpha)q-gekoppelten Rezeptoren die Rezeptoren Muscarin-M1, -M3, -M5, Serotonin-5HT2, Bombesin, Cholecystokinin, Neurokinin, Prostaglandin-E-Typ I, Adenosin-A2B, P2Y1, P2Y2, P2Y4, P2Y6, P2Y11, Calcitonin, mGluR1-Rezeptoren, Angiotensin II Rezeptor 1, "Protease Activated Receptor 1" und Serotonin R genannt werden. Selbstverständlich können auch, wie vorstehend dargelegt, funktionelle Derivate, Allele, Fragmente oder Mutanten dieser Rezeptoren verwendet werden.

Aufgrund der oben dargelegten Freiheit bei der Auswahl der im erfindungsgemä-ßen Verfahren zu verwendenden bzw. zu untersuchenden Rezeptoren besteht auch keinerlei Einschränkung hinsichtlich der jeweils einzusetzenden Agonisten bzw. Antagonisten. Ein "Agonist" ist erfindungsgemäß eine Substanz, die den jeweiligen Rezeptor aktiviert, während ein "Antagonist" die Aktivierung des jeweiligen Rezeptors inhibiert, insbesondere aufgrund seiner Struktur an eine inaktive Konformation des Rezeptors angepaßt ist. In den erfindungsgemäßen Verfahren einsetzbare Agonisten und Antagonisten der jeweiligen Rezeptoren können somit aus allen möglichen Substanzklassen ausgewählt sein. Zu nennen sind bspw. anorganische Moleküle, z.B. auch mit Rezeptoren wechselwirkende lonen, kleine organische Verbindungen sowie auch agonistisch oder antagonistisch auf den jeweiligen Rezeptor wirkende Biomoleküle, insbesondere Peptide, Polypeptide, bspw. Proteine, Nukleinsäuren, Lipide oder Saccharide, wie Mono-, Oligo- oder Polysaccharide. Ebenfalls können Kombinationen aus derartigen Molekülen, die synthetisch oder natürlich vorkommend sein können, Agonisten oder Antagonisten der in Rede stehenden Rezeptoren sein. Als weitere Beispiele von Agonisten G(alpha)q-gekoppelter Rezeptoren können Nukleobasen bzw. Nukleotide, wie z.B. Adenosin, AMP, ADP, ATP, Uridine, UMP, UDP, UTP, Cytosin, CMP, CDP, CTP, Guanosin, GMP, GDP, GTP, Thymidin, TMP, TDP, TTP, lonsin, IMP, IDP und ITP sowie Muscarin-Rezeptoragonisten (bspw. Muscarin, Bethanechol, Metocloprimid, Pilocarpin und Oxotremorin M) genannt werden. Beispiele von Agonisten von G(alpha)i- und/oder G(alpha)o-gekoppelten Rezeptoren sind die bekannten Agonisten der obigen Rezeptoren, wie GRT0777S, Damgo, Fentanyl, Serotonin, Morphine, wie Morphin, Buprenorphin usw. Antagonisten der genannten G(alpha)i- bzw. G(alpha)o-gekoppelten Rezeptoren sind ebenfalls einem Fachmann bekannt. Ein spezifisches Beispiel ist Naloxon.

Die jeweilige beim entsprechenden Verfahren einzusetzende Kombination von Agonisten und ggf. Antagonisten hängt von den jeweiligen Rezeptoren ab, wobei unter anderem die endogene Ausstattung der jeweils verwendeten Zellen mit Rezeptoren der in Rede stehenden Klassen bzw. mit anderen Rezeptoren, welche aber möglicherweise den oder die zur Verwendung ins Auge gefassten Agonisten oder ggf. Antagonisten ebenfalls binden, ab. Soll ein bestimmter Agonist oder ggf. Antagonist eines bestimmten G(alpha)q-gekoppelten Rezeptors bzw. eines G(alpha)i/o-gekoppelten Rezeptors verwendet werden, so sollte dieser Agonist oder dieser Antagonist vorzugsweise in Kombination mit solchen Zellen eingesetzt werden, die keinen Rezeptor exprimieren, der diesen Agonisten oder Antagonisten ebenfalls bindet, bzw. die Zellen sollten vorzugsweise nur solche anderen Rezeptoren exprimieren, die den in Rede stehenden Agonisten oder Antagonisten deutlich schwächer binden, d.h. eine deutlich geringere Affinität zu diesem Agonisten bzw. Antagonisten aufweisen. Vorzugsweise sollte die Affinität für den jeweiligen Agonisten bzw. Antagonisten, der in den erfindungsgemäßen Verfahren eingesetzt werden soll, bezüglich anderer Rezeptoren im Vergleich zu den in Rede stehenden G(alpha)q-gekoppelten bzw. G(alpha)i/o-gekoppelten Rezeptoren mindestens um einen Faktor von etwa 3, mehr bevorzugt mindestens um einen Faktor von mindestens etwa 10, insbesondere um einen Faktor von wenigstens etwa 20 niedriger sein. Dies gilt selbstverständlich auch für den Fall, daß der zur Verwendung vorgesehene Agonist des G(alpha)q-gekoppelten Rezeptors auch vom zu untersuchenden G(alpha)i/o-gekoppelten Rezeptor gebunden wird und umgekehrt. Diese Gesichtspunkte gelten selbstverständlich auch hinsichtlich des vorstehend definierten Antagonisten von G(alpha)i/o-gekoppelten Rezeptoren in Hinblick auf eine mögliche Wechselwirkung mit dem G(alpha)q-gekoppelten Rezeptor.

Auch hinsichtlich der in den erfindungsgemäßen Verfahren einzusetzenden Zellen besteht keinerlei Einschränkung, solange gewährleistet ist, daß die Zellen neben dem G(alpha)i/o-gekoppelten Rezeptor auch mindestens einen G(alpha)q-gekoppelten Rezeptor exprimieren. Somit können Zellen von stabilen Zellinien, primären Zellkulturen oder auch Gewebezellen in den erfindungsgemäßen Verfahren eingesetzt werden. Zellinien weisen dabei den Vorteil auf, daß sie nötigenfalls genetisch manipuliert werden können, um bspw. für eine exogene Expression eines bestimmten Rezeptors zu sorgen oder aber, falls die ins Auge gefaßte Zellinie einen oder mehrere Rezeptoren endogen oder auch exogen aufgrund einer vorherigen stabilen Transfektion exprimiert, welcher bei der zu verwendenden Kombination aus Agonisten des G(alpha)q-gekoppelten Rezeptors und des G(alpha)i/o-gekoppelten Rezeptors bzw. des Antagonisten letzterer Rezeptoren stören könnte, die Expression derartiger störender Rezeptoren durch einen Fachmann bekannte Maßnahmen, bspw. Verhinderung von Translation oder Transkription, bspw. mittels Antisense-Techniken oder auch RNA-Interferenz-Techniken oder durch Knockout-Verfahren zu unterdrücken bzw. zu verhindern. Daher ist es erfindungsgemäß möglich, durch die einem Fachmann bekannten, insbesondere gentechnischen Verfahren möglich, ein spezifisches Meß- bzw. Testsystem bereitzustellen, das für die ins Auge gefaßte Verwendung als Verfahren zur Messung der Aktivierung oder Inaktivierung oder zur Identifizierung von Agonisten oder Antagonisten G(alpha)i/o-gekoppelter Rezeptoren maßgeschneidert ist.

Als bevorzugte Beispiele erfindungsgemäß einsetzbarer Zellinien können jegliche eukaryotischen Zellinien, insbesondere Säugerzellinien, angegeben werden, bspw. CHO (z.B. CHO K1), HEK293, THP-1, SH-SY5Y, Jurkat, HeLa, L-Zellen, A-10, Cos-7, NIH 3T3, ECV304, RBL, UMR 106, PC3 GH3, PC1 und IMR-32. Dabei werden nach einer weiteren bevorzugten Ausgestaltung der erfindungsgemäßen Verfahren aufgrund der jeweiligen endogenen Expression eines oder mehrerer spezifischer G(alpha)q-gekoppelter Rezeptoren bestimmte Kombinationen von Zellinie/Agonist des/der G(alpha)q-gekoppelten Rezeptors/Rezeptoren bevorzugt eingesetzt. Bspw. wird bei HEK293-Zellen bevorzugt ATP (zur untergrenzwertigen Stimulation der P2Y1- oder P2Y2-Rezeptoren), Muscarin (zur untergrenzwertigen Stimulation des M1-Rezeptors) und/oder Somatostatin (zur untergrenzwertigen Stimulation des Somatostatin-Rezeptors) verwendet, um eine untergrenzwertige Stimulation des oder der jeweiligen G(alpha)q-gekoppelten Rezeptors/Rezeptoren zu bewirken. In ähnlicher Weise wird bei CHO-K1-Zellen bevorzugt ATP und/oder UTP verwendet, um eine untergrenzwertige Stimulation des endogen in diesen Zellen vorhandenen P2Y2-Rezeptors als G(alpha)q-gekoppelten Rezeptor hervorzurufen. Die vorstehenden Sachverhalte gelten bei Stimulation endogen exprimierter Rezeptoren. Selbstverständlich ist es jedoch einem Fachmann unter Verwendung molekular-/zellbiologischer Standardtechniken grundsätzlich möglich, irgendeine gewünschte Kombination von Rezeptoren in einer gewählten Zelle heterolog zu exprimieren (eventuell unter Ausschalten bzw. Herrunterregulation der Expression eines oder mehrerer endogen vorhandener, möglicherweise sonst störender Rezeptoren), um so eine für die jeweiligen Bedürfnisse maßgeschneiderte Kombination von Rezeptor(en)/Rezeptor-Agonist(en) verwenden zu können.

Die Messung der cytoplasmatischen Ca²⁺-Konzentration kann bei Verwendung von Ca²⁺-abhängigen Farbstoffen, insbesondere von Fluoreszenzfarbstoffen, in einfacher Weise fotometrisch erfolgen. Zu diesem Zweck sind im Handel verschiedene Meßvorrichtungen erhältlich, bspw. Fluoroskan^{®} (Labsystems) oder FLIPR^{®} (Molecular Devices). Mit Hilfe derartiger Meßsysteme und des weiteren unter Verwendung von geeigneten, einem Fachmann bekannten Pepetiereinrichtungen, Kulturvorrichtungen, HPLC-Komponenten, insbesondere Pumpen, Ventile usw. sowie unter Zuhilfenahme einem Fachmann bekannter Datenverarbeitungsanlagen und -programmen, insbesondere zur Erfassung, Speicherung und Auswertung der Meßwerte, können die erfindungsgemäßen Verfahren selbstverständlich halb- oder vollautomatisch ausgestaltet werden und darüber hinaus auch mit den unterschiedlichsten Durchsatzgeschwindigkeiten ausgeführt werden, weshalb bspw. bei den erfindungsgemäßen Meß- und Identifizierungsverfahren auch Hochdurchsatz- (engi."high throughput screening", HTS-) Applikationen möglich sind.

Ein bevorzugtes Anwendungsgebiet der erfindungsgemäßen Verfahren ist die Messung der Deaktivierung/Aktivierung von G(alpha)i/o-gekoppelten Rezeptoren, die im Zusammenhang mit der Signalentstehung, - weiterleitung und/oder - verarbeitung von Schmerzempfindungen beteiligt sind. Demgemäß dient das erfindungsgemäße Identifizierungsverfahren für Agonisten bzw. Antagonisten von G(alpha)i/o-gekoppelten Rezeptoren bspw. dem Screening von Testsubstanzen bei G(alpha)i/o-gekoppelten Rezeptoren, die im Zusammenhang mit Schmerzempfindungen stehen. Weitere Anwendungsgebiete sind das Screening von Testsubstanzen bei G(alpha)i/o-gekoppelten Rezeptoren, die mit immunologischen Fehlfunktionen bzw. Erkrankungen (hier bspw. die Rezeptoren CXCR, CCR, XCR1 und CX3R) und neurodegenerativen Erkrankungen, z.B. Parkinson (hier z.B. die Rezeptoren der Dopamin-Familie) in Zusammenhang stehen.

Die Figuren zeigen:

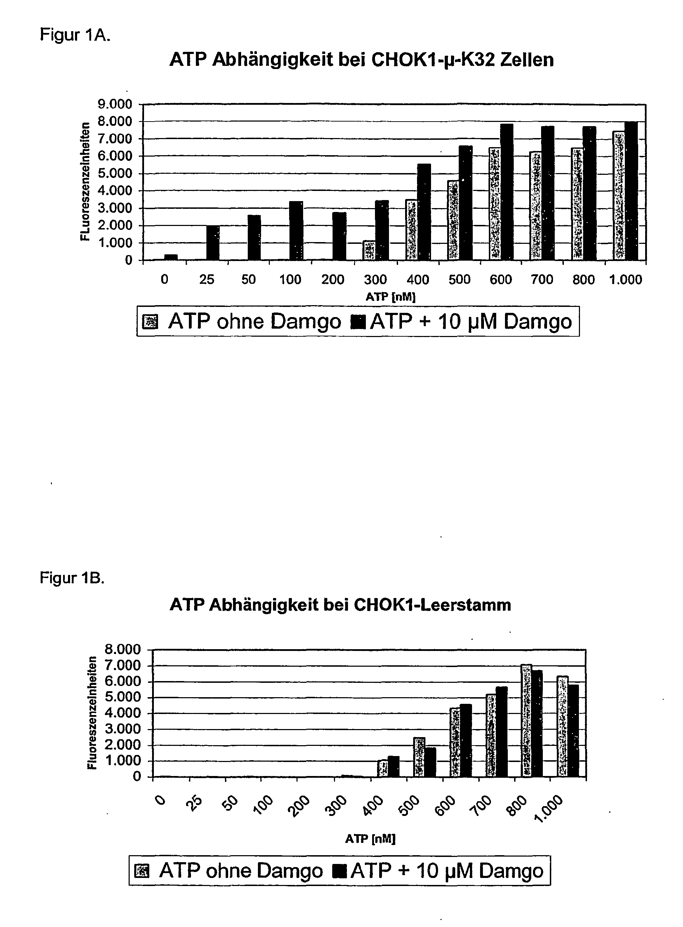

In den Fig. 1 bis 6 ist jeweils die relative Fluoreszenzintensität in Abhängigkeit von der Konzentration bestimmter Agonisten bzw. Antagonisten G-gekoppelter Zellrezeptoren aufgetragen. Die relative Fluoreszenzintensität ist ein direktes Maß für die Ca²⁺-Konzentration aufgrund der Verwendung eines Ca²⁺-abhängigen Fluoreszenzfarbstoffs (Fluo-4).

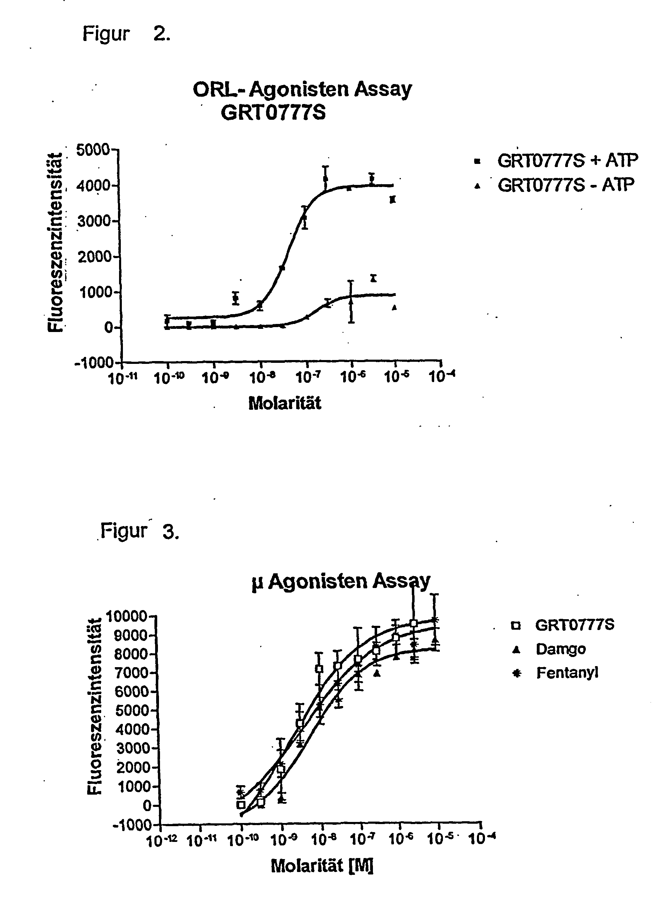

- Fig. 1A: zeigt die Fluoreszenzintensität (als relative Einheit) bei der Zellinie CHOK1-µ-K32 bei verschiedenen ATP-Konzentrationen im Bereich von 0 bis 400 nM. Es zeigt sich in der Abwesenheit von DAMGO (graue Balken), einem µ-Opioid-Rezeptor-Agonist, daß erst bei Zusatz von 300 nM ATP ein deutliches Fluoreszenzsignal von mehr als 1.000 relativen Einheiten gemessen wird, während bei 100 nM die gemessene Fluoreszenzintensität um mehr als einen Faktor 100 kleiner ist. ln der Anwesenheit von 10 µM DAMGO (schwarze Balken), aber ohne ATP, wird ein relativ kleines Fluoreszenzsignal von ca. 300 relativen Einheiten beobachtet. Dieses Signal wird in Gegenwart von 25 nM ATP wesentlich verstärkt. Bei diesen ATP-Konzentrationen ergab sich, wie oben dargelegt, nur ein sehr geringes Fluoreszenzsignal, wenn kein DAMGO vorhanden war. Bei steigenden Konzentrationen von ATP (300 nM und mehr) sinkt die Stärke des von DAMGO ausgelösten Signals, da ATP allein bei diesen Konzentrationen dazu befähigt ist, vergleichbare Signalstärken hervorzurufen. Wie ein Vergleich der Balken für 100 nM ATP in der Fig. 1A zeigt, ergibt sich bei dieser ATP-Konzentration die größte Signalverstärkung infolge der Wirkung von DAMGO. Daher wurde diese ATP-Konzentration (100 nm) in den Experimenten, deren Ergebnisse gemäß den Fig. 2 bis 6 beschrieben sind, verwendet.
- Fig. 1 B: zeigt die Fluoreszenzintensität bei der Zellinie CHOK1-Leerstamm unter die gleiche Bedingungen wie Fig. 1A. Ab einer Konzentration von 300 nM löst ATP durch den endgogen P2Y2-Rezeptor ein Fluoreszenzsignal aus, daß durch DAMGO nicht beeinflußt wird. Ein Fluoreszenzsignal im untergrenzwertigen Bereich ist nicht vorhanden, da der µ-Opioid-Rezeptor in diesen Zellen fehlt und ein Synergismus nicht zustande kommen kann.
- Fig. 2: zeigt, daß die Gegenwart des Agonisten des G(alpha)q-gekoppelten Rezeptors (ATP) zur Erzeugung eines deutlichen Fluoreszenzsignals erforderlich ist. Es wurden in diesem Fall stabil ORL1 exprimierende Zellen (Zellinie CHOK1-ORL-K66) verwendet, jedoch sind die Ergebnisse für den µ-Rezeptor exprimierende Zellen unter den gleichen Bedingungen ähnlich. GRT0777S erzeugt ein schwaches Signal bei ansteigender Konzentration in Abwesenheit von ATP (▲). In Gegenwart von 100 nM ATP ist das meßbare Signal bei GRT0777SKonzentrationen von mehr als 10⁻⁹ M deutlich verstärkt (■). Darüber hinaus ist die Variabilität der Meßergebnisse (vgl. die für jeden Meßpunkt in der Fig. angegebenen Abweichungen) zwischen unabhängigen Experimenten unter den gleichen Bedingungen geringer, wenn GRT0777S in Gegenwart von 100 nM ATP verwendet wird. Somit kann die Aktivierung des G(alpha)i/o-gekoppelten Rezeptors durch seinen Agonisten in Gegenwart der geeigneten untergrenzwertigen Konzentration des Agonisten eines G(alpha)q-gekoppelten Rezeptors zuverlässig gemessen werden. Des weiteren erlaubt die erhöhte Signalstärke auch die quantitative Erfassung der Rezeptoraktivierung im suboptimalen Bereich, was zu einer fünffachen Verminderung des geschätzten IC50-Werts (4,2 x 10⁻⁸ M statt 1,7 x 10⁻⁷ M) führt. Der mit Hilfe des erfindungsgemäßen Verfahrens ermittelte IC50-Wert von 4,2 x 10⁻⁸ M liegt somit deutlich näher an Daten, die mittels Rezeptorbindungsstudien ermittelt wurden (Ki = 4,0 x 10⁻⁹)
- Fig. 3: zeigt die Fluoreszenzintensität von CHOK1-µ-K32-Zellen in Abhängigkeit von der zugesetzten Konzentration der µ-Rezeptoragonisten GRT0777S (□), Damgo (▲) und Fentanyl (*), jeweils in Gegenwart von 100 nM ATP. Dargestellt sind die Durchschnittswerte von jeweils 3 unabhängigen Messungen, wobei die Standardabweichungen angezeigt sind. Es zeigen sich für alle drei Rezeptor-Agonisten klassische Dosis-Antwort-Kurven. Die sehr ähnlichen Meßwerte aller drei Rezeptor-Agonisten ist darauf zurückzuführen, daß alle drei Substanzen ähnliche Bindungsaffinitäten zum µ-Opioid-Rezeptor aufweisen (Ki zwischen 16 und 24 nM). Wenn der gleiche Test bei nicht transfizierten CHOK1-Zellen durchgeführt wurde, ergab keine der verwendeten µ-Rezeptoragonisten ein Fluoreszenzsignal oberhalb des Hintergrunds (nicht gezeigt).

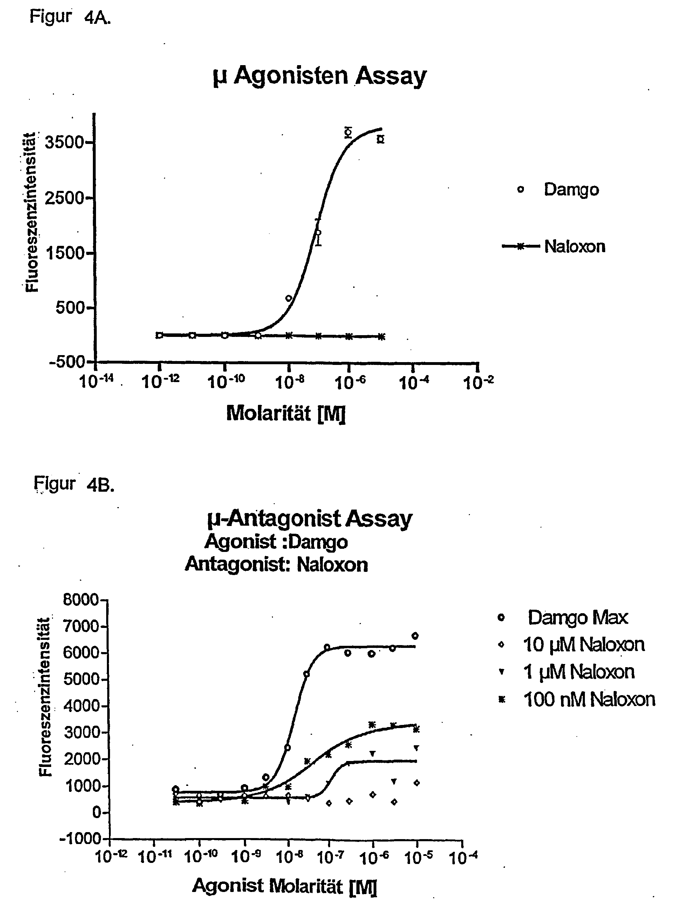

- Fig. 4A: zeigt die Fluoreszenzintensität bei CHOK1-µ-K32 (Exprimieren den µ-Opioid-Rezeptor) in Abhängigkeit unterschiedlicher Konzentrationen des µ-Agonisten Damgo (○) bzw. des µ-Antagonisten Naloxon (*), jeweils in Gegenwart von 100 nM ATP. Während Damgo, wie schon in den Experimenten gemäß Fig. 3 festgestellt, eine klassische Dosis-Antwort-Kurve der Fluoreszenzintensität ergibt, kann erwartungsgemäß Naloxon keine Fluoreszenzintensität oberhalb des Hintergrunds erzeugen.
- Fig. 4B: zeigt in einem der Fig. 4A entsprechenden Experiment die Fluoreszenzintensität in Abhängigkeit von der zugesetzten DamgoKonzentration in Gegenwart von 100 nM ATP, jeweils für unterschiedliche Konzentrationen des µ-Rezeptorantagonisten Naloxon. Das Experiment wurde ohne Naloxon (○), in Gegenwart von 100 nM Naloxon (*), 1 µM Naloxon (▼) und 10 µM Naloxon (◇) durchgeführt. Wenn sowohl Damgo als auch Naloxon zusammen zugesetzt wurden (in Gegenwart von 100 nM ATP), wird das von Damgo erzeugte Signal Dosis-abhängig von dem Antagonisten Naloxon vermindert. Daher kann das erfindungsgemäße Verfahren nicht nur zur Detektion von Agonisten , sondern auch von Antagonisten bezüglich eines gegebenen G(alpha)i/o-gekoppelten Rezeptor verwendet werden. Des weiteren erlaubt der große dynamische Bereich des Tests die Detektion selbst relativ schwacher Antagonisten.

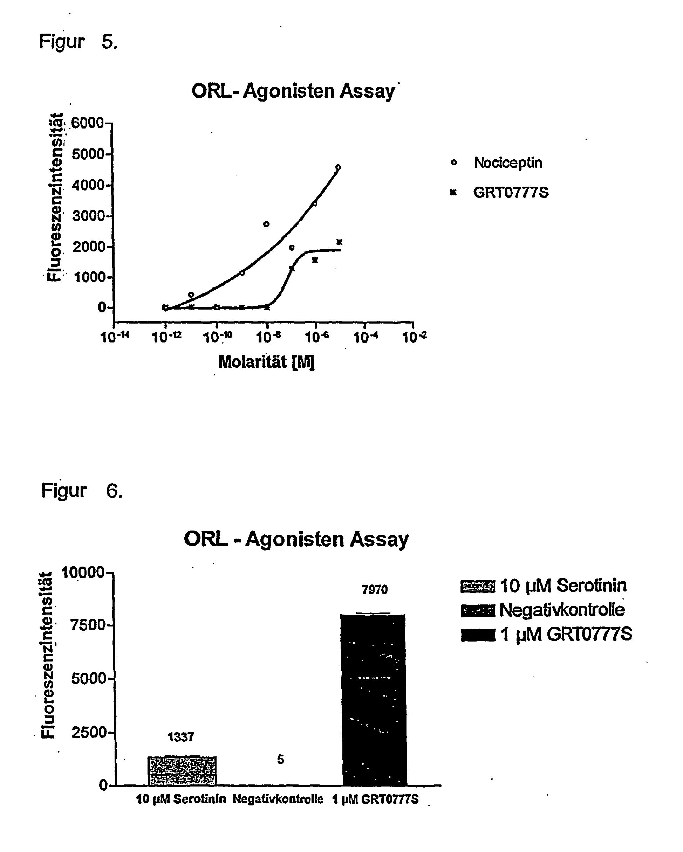

- Fig. 5: zeigt die Fluoreszenzintensität bei Zellen der Zellinie CHOK1-ORL-K66, welche den ORL1-Opioid-Rezeptor stabil exprimieren. Die Zellen wurden entweder mit unterschiedlichen Konzentrationen an GRT0777S (*) oder Nociceptin (○) Jeweils in Gegenwart von 100 nM ATP behandelt. Sowohl GRT0777S als auch das Peptid Nociceptin (Ophanin FQ) zeigen ihre agonistischen Eigenschaften gegenüber dem ORL1-Rezeptor, während nicht transfizierte Zellen kein Signal erzeugen (nicht gezeigt). Daher ist das dem erfindungsgemäßen Test zugrunde liegend synergistische Prinzip zwischen G(alpha)q-gekoppeltem Rezeptor und G(alpha)i/o-gekoppelten Rezeptoren unabhängig vom jeweiligen Rezeptor, da, wie in den Figuren 1 bis 4 gezeigt, die Signalverstärkung sowohl beim µ-Opioid-Rezeptor als auch (Fig. 5) beim ORL-1-Opioid-Rezeptor festgestellt wird.
- Fig. 6: belegt, daß das der vorliegenden Erfindung zugrunde liegende Meßprinzip auch zur Untersuchung endogen exprimierter G(alpha)i/o-gekoppelter Rezeptoren eingesetzt werden kann. Dazu wurden CHO-Zellen, die den ORL1-Rezeptor überexprimieren, als Positivkontrolle verwendet. Zellen, die mit ATP allein behandelt werden, zeigen kein signifikantes Signal (Negativkontrolle). Mit 1 µM GRT0777S behandelte Zellen zeigen demgegenüber eine deutlich erhöhte Ca²⁺-Konzentration, wie die mittlere Fluoreszenzintensität von 7970 relativen Einheiten zeigt. CHO-Zellen exprimieren jedoch endogen einen weiteren G(alpha)i/o-Rezeptor, nämlich den 5HT1 B-Rezeptor. Werden die Zellen mit Serotonin, dem natürlichen Liganden des endogen von den CHO-Zellen exprimierten 5HT-1 B-Rezeptor in Gegenwart von 100 nM ATP behandelt (Serotonin-Konzentration: 10 uM) wird immerhin eine Fluoreszenzintensität von durchschnittlich 1337 relativen Einheiten und damit ein deutliches Signal gemessen. Die in der Fig. 6 dargestellten Messergebnisse stellen jeweils den Mittelwert von vier unabhängigen Messungen dar. Gegenüber dem von GRT0777S erzeugten Signal ist die von Serotonin hervorgerufene Ca²⁺-Konzentration zwar relativ gering. Dies wurde jedoch im vorliegenden Vergleichsexperiment erwartet, da der von den CHOK1-ORL-K66-Zellen exprimierte ORL1-Rezeptor in sehr viel größerer Kopienzahl als der endogen exprimierte Serotonin-Rezeptor vorliegt. Eine ähnliche Aktivierung durch Serotonin wird bei nicht-transfizierten CHOK1-Zellen beobachtet, während bei diesen, mangels eines exprimierten ORL1-Rezeptors, GRT0777S kein Signal mehr auszulösen vermag (nicht gezeigt).

Zusammenfassend konnte durch die in den vorstehenden Figuren dargelegten Experimente gezeigt werden, daß die Kombination eines G(alpha)q-abhängigen Stimulus, hier ATP bezüglich des P2Y2-Rezeptors, mit einer Stimulierung eines G(alpha)i/o-gekoppelten Rezeptor die empfindliche und genaue Messung der Aktivierung des in Rede stehenden G(alpha)i/o-gekoppelten Rezeptors durch einen Agonisten dieses Rezeptors oder auch die Verhinderung der Aktivierung eines G(alpha)i/o-Rezeptors aufgrund der Wirkung eines Antagonisten dieses Rezeptors erlaubt. Es wurde gezeigt, daß das erfindungsgemäße Meßprinzip die Untersuchung artifiziell exprimierter Rezeptoren, hier der µ-Opioid-Rezeptor und der ORL1-Rezeptor, als auch die Untersuchung endogen exprimierter Rezeptoren, hier der 5HT-1B-Rezeptor, ermöglicht.

Die folgenden Ausführungsbeispiele erläutern die vorliegende Erfindung näher, ohne sie einzuschränken.

### 1. Ausführungsbeispiel

### Materialien

Für sämtliche Ausführungsbeispiele wurden folgende Materialien verwendet.

Biocoat PDL 96-Well-Platten Black Nr. 356640 von Becton-Dickinson 75 cm² Flaschen von Sarstedt.
DMSO: Sigma
Fluo-4-AM-ester: Molecular Probes F14201
Probenecid: Molecular Probes
HBSS Puffer: Invitrogen
HEPES: Sigma
Zeocin (zur Selektion der Zellen): Invitrogen
Zellkulturmedium: Ham's F12 (Gibco Nr.: 21765029), 10 % FCS (PAA), 18 µg /ml L-Prolin (Sigma), 180 µg/ml Zeocin (Invitrogen)
HBSS 1 x ohne Phenolrot (Gibco-Nr.: 140254-050), 2,5 mM Probenicid (Molecular Probes), 20 mM HEPES (Sigma)

### Cytoplasmatische Ca²⁺-Messung

Die Messung der cytoplasmatischen Ca2+-Konzentration erfolgte bei allen Ausführungsbeispielen unter Verwendung des FLIPR^{®} Calcium Plus Assay Kits und einem entsprechenden FLIPR^{®}-Gerät von Molecular Devices nach den Angaben des Herstellers.

Kurz dargestellt, wurden die die zu messenden Zellen am Vortag des Experiments in einer Dichte von 25.000 Zellen pro Vertiefung einer geeignete Zellkultur-Platte mit 96 Vertiefungen ("Wells") in einem Volumen von 100 µl des entsprechenden Mediums ausplattiert. Für den Test wurden mit Poly-D-Lysin beschichtete Zellkultur-Platten der Firma Becton-Dickinson (Best. Nr. 66440) verwendet. Am Versuchstag wurden die Zellen mit Fluo-4 beladen. Dazu wurden ein Vial Fluo-4 in 23 µl DMSO sowie 23 µl Pluronic F 127 aufgelöst. Davon wurden 42 µl in 21 ml HBSS (+ Probenecid und Hepes) gegeben. Von dieser Lösung wurden 50 µl pro Vertiefung auf die Zellen gegeben, wobei die Platten zur Beladung für 30 min bei 37°C, 5% Co₂, 98% relativer Luftfeuchte, inkubiert wurden. Um die überschüssige Beladungslösung zu entfernen, wurden die Platten in einem Wsachgerät für Platten mit 96 Vertiefungen dreimal mit 200 µl HBSS gewaschen. Daran schloss sich ein Dispensionsschritt an, in dem jeweils 100 µl HBSS auf die Zellen gegeben wurden. Die Platten wurden danach für mindestens 15 min bei Raumtemperatur im Dunkeln stehen gelassen.

Nachdem das FLIPR^{®}-Gerät ca. ½ h vor Versuchsbeginn eingeschaltet wurde, wurde das entsprechende FLIPR^{®}-Programm durchgeführt.

Es wird dabei zwischen Agonisten- (eine Zugabe) sowie Antagonisten-Test (zwei Zugaben) unterschieden. Die zu testenden Substanzen lagen entsprechend der Verdünnung im Test höher konzentriert vor. Je nach Test wurden die "Cell-Plates" und "Drug-Plates" in die Vorratsposition oder in die entsprechende Messposition im FLIPR^{®} gebracht. Vor jeder Messung wurde die Beladung der Zellen durch ein Kurzmessung mit der entsprechenden "Snapshot"-Funktion überprüft. Dann wurde das Programm für die eigentliche Messung (vorliegend Excitation bei 488 nm, Emission bei 510 bis 570 nm) gestartet.

Die Kalibrierung der FLIPR^{®}-Messvorrichtung erfolgte 1x wöchentlich mit einer Kalibrierungsplatte.

### Bestimmung der zur untergrenzwertigen Aktivität eines G(alpha)q-gekoppelten Rezeptors erforderliche Menge eines Agonisten

Als Modellsystem für die Durchführung des erfindungsgemäßen Messprinzips wurden CHO K1-Zellen verwendet, die zur Expression von G(alpha)i- bzw. G(alpha)o-gekoppelter Opioid-Rezeptoren (µ- oder ORL1-Rezeptor) mit entsprechenden Konstrukten unter Verwendung von Lipofektamin 2000 stabil transfiziert wurden. CHO K1-Zellen exprimieren endogen zwei Rezeptoren für ATP, P2Y2 und P2X7. P2Y2 ist ein G(alpha)q-gekoppelter Rezeptor, dessen Affinität für ATP und UTP vergleichbar ist. P2X7 ist ein nicht-selektiver lonenkanal, der UTP nicht bindet. Dieser Rezeptor bindet ATP etwa 100fach schwächer als der G(alpha)q-gekoppelte Rezeptor P2Y2. Daher sind die vorliegend gemessenen Signale (Ca²⁺-Konzentration) bei den betrachteten Konzentrationsbereichen des Rezeptoragonisten ausschließlich vom P2Y2-Rezeptor abhängig.

Um die zur Messung der Aktivität der exogen experimierten Opioid-Rezeptoren geeignete Menge bzw. Konzentration von ATP zu bestimmen, wurden Fluoreszenz-Messreihen mit CHO-µ-K32-Zellen (stabile Expression des µ-Opioid-Rezeptors) und unterschiedlichen ATP-Konzentrationen durchgeführt (Fig. 1A, helle Balken, ATP ohne DAMGO). Erst ab eine Konzentration von 300 nM ATP allein ist eine Änderung der Fluoreszenz detektierbar.

Das Experiment wurde mit den gleichen ATP-Konzentrationen, jedoch in Gegenwart von DAMGO, einem Agonisten des µ-Opioid-Rezeptors, wiederholt (Fig. 1A, dunkle Balken, ATP + 10 µM DAMGO). Bei 100 nM ATP ergab sich die größte Signalverstärkung. Daher wurden die folgenden Experimente bei dieser ATP-Konzentration durchgeführt.

Das gleiche Experiment wurde an dem CHO-K1 Zellen, ohne den stabil transfizierten µ-Opioid-Rezeptor (sogenannte CHOK1-Leerstamm), durchgeführt (Fig. 1B). In der Abwesenheit des µ-Opioid-Rezeptors wurde keine Signalverstärkung im untergrenzwertigen Bereich beobachtet, was die Notwendigkeit des µ-Opioid-Rezeptors für die synergistischen Effekte nachweist. Ohne einen entsprechenden Rezeptor hat DAMGO keinen signifikanten Effekt auf das ATP-verursachte Signal. Hierdurch wurde gezeigt, daß kein endogener Rezeptor die DAMGO-abhängigen Effekte vermittelt.

### 2. Ausführungsbeispiel

### Messung der Aktivierung G(alpha)ilo-gekoppelter Rezeptoren durch unterschiedliche Agonisten

Es wurde die Aktivierung des µ-Rezeptors durch die Agonisten GRT0777S, Damgo und Fentanyl gemessen, wobei sich typische Dosis-Antwort-Kurven im gemessenen Konzenttionsbereich (10 µM bis 0,1 nM) ergaben (Fig. 3).

Die Aktivierung des ORL1-Rezeptors bei entsprechend stabil transfizierten CHO K1-Zellen (CHOK1-ORL-K66) durch GRT0777S (Fig. 2) bzw. durch den Peptidantagonisten Nociceptin/Ophanin FQ (Fig. 5) (jeweils in Gegenwart von 100 nM ATP) ergab die erwarteten Dosis-Antwort-Kurven bei den mittels Fluoreszenz gemessenen Ca²⁺-Konzentrationen, während ein Vergleichstest mit GRT0777S ohne ATP-Zusatz (Fig. 2) nur ein sehr schwaches Signal hervorrief. Das erfindungsgemäße Messprinzip ist daher vom jeweiligen Rezeptor bzw. Agonisten unabhängig. Die Gegenwart des Agonisten des G(alpha)q-gekoppelten Rezeptors ist hingegen erforderlich.

### 3. Ausführungsbeispiel

### Messung der Deaktivierung G(alpha)ilo-gekoppelter Rezeptoren durch Antagonisten

Das erfindungsgemäße Messprinzip eignet sich auch zur Bestimmung der Hemmung der Aktivierung G(alpha)i/o-gekoppelter Rezeptoren durch entsprechende Antagonisten.

Im Falle des µ-Opioid-Rezeptors wurden dazu stabil diesen Rezeptor exprimierende CHO K1-Zellen (CHOK1-µ-K32) zunächst mit dem Rezeptoragonisten Damgo und 100 nM ATP bzw. mit dem Rezeptorantagonisten Naloxon und 100 nM ATP behandelt (Fig. 4A). Damgo zeigte die erwartete Dosis-Antwort-Kurve, während Naloxon allein kein Signal erzeugte.

Wurde jedoch Damgo zusammen mit Naloxon eingesetzt, so verminderte sich das von Damgo hervorgerrufene Signal in Abhängigkeit von der Naloxon-Konzentration, hier über einen Bereich von drei Größenordnungen, 100 nM bis 10 µM Naloxon (Fig. 4B).

### 4. Ausführungsbeispiel

### Messung der Aktivierung endogen exprimierter G(alpha)ilo-gekoppelter Rezeptoren

Da CHO K1-Zellen endogen nicht nur den G(alpha)q-gekoppelten ATP- (bzw. UTP-) Rezeptor P2Y2 sondern auch den G(alpha)i/o-gekoppelten Serotonin-Rezeptor 5HT-1 B exprimieren, wurden diese Zellen (CHOKI-ORL-K66) verwendet, um zu zeigen, daß das synergistische Messprinzip der vorliegenden Erfindung auch zur Messung der Aktivierung endogen exprimierter Rezeptoren geeignet ist.

Dazu wurden die Zellen nur mit 100 nM ATP (Negativkontrolle), 100 nM ATP plus 1 µM GRT0777S (Positivkontrolle) und 100 nM ATP plus 1 µM Serotonin behandelt. Gegenüber der Negativkontrolle ergab die Zugabe des 5HT-1 B-Agonisten Serotonin eine deutliche Signalverstärkung bei der Fluoreszenzmessung (Fig. 6).

## Patentansprüche

1. In vitro Verfahren zur Messung der Aktivierung eines G(alpha)i- und/oder G(alpha)o-gekoppelten Rezeptors bei Zellen, die mindestens einen G(alpha)q-gekoppelten Rezeptor exprimieren, umfassend die Schritte:
(a) Gleichzeitiges Behandeln der Zellen mit einer derartigen Menge eines Agonisten des G(alpha)q-gekoppelten Rezeptors, daß sich eine untergrenzwertige Aktivität ergibt, und mit einem Agonisten des G(alpha)i- und/oder G(alpha)o-gekoppelten Rezeptors; und
(b) Messen der cytoplasmatischen Ca²⁺-Konzentration der Zellen,
wobei die Menge des Agonisten des G(alpha)q-gekoppelten Rezeptors, die eine untergrenzwertige Aktivität ergibt, diejenige ist, bei welcher das Verhältnis des Ergebnisses der Messung der cytoplasmatischen Ca²⁺-Konzentration der Zellen bei deren Behandlung mit einer Menge des Agonisten des G(alpha)q-gekoppelten Rezeptors zum Ergebnis der Messung der cytoplasmatischen Ca²⁺-Konzentration der Zellen bei deren Behandlung mit der gleichen Menge des Agonisten des G(alpha)q-gekoppelten Rezeptors und gleichzeitiger Behandlung der Zellen mit einer Menge eines Agonisten des G(alpha)i- und/oder G(alpha)o-gekoppelten Rezeptors, die zur vollständigen Aktivierung ausreicht, nicht größer als 1:3 ist.

2. In vitro Verfahren zur Identifizierung von Agonisten eines G(alpha)i- und/oder G(alpha)o-gekoppelten Rezeptors, umfassend die Schritte:
(i) Bereitstellen von Zellen, die den G(alpha)i- und/oder G(alpha)o-gekoppelten Rezeptor und mindestens einen G(alpha)q-gekoppelten Rezeptor exprimieren; und
(ii) Durchführen der Schritte (a) und (b) gemäß dem Verfahren nach Anspruch 1, wobei im Schritt (a) als Agonist des G(alpha)i- und/oder G(alpha)o-gekoppelten Rezeptors eine Testsubstanz, deren Aktivität bezüglich des G(alpha)i- und/oder G(alpha)o-gekoppelten Rezeptors getestet werden soll, verwendet wird.

3. In vitro Verfahren zur Messung der Deaktivierung eines G(alpha)i- und/oder G(alpha)o-gekoppelten Rezeptors bei Zellen, die mindestens einen G(alpha)q-gekoppelten Rezeptor exprimieren, umfassend die Schritte:
(A) Gleichzeitiges Behandeln der Zellen mit einer Menge eines Agonisten des G(alpha)q-gekoppelten Rezeptors derart, daß sich eine untergrenzwertige Aktivität ergibt, und mit einer Menge eines Agonisten des G(alpha)i- und/oder G(alpha)o-gekoppelten Rezeptors, die gerade zur vollständigen Aktivierung ausreicht;
(B) Messen der cytoplasmatischen Ca²⁺-Konzentration der Zellen;
(C)Gleichzeitiges Behandeln der Zellen mit der gleichen Menge von Schritt (A) des Agonisten des G(alpha)q-gekoppelten Rezeptors, mit der gleichen Menge von Schritt (A) des Agonisten des G(alpha)i- und/oder G(alpha)o-gekoppelten Rezeptors und mit einem Antagonisten des G(alpha)i- und/oder G(alpha)o-gekoppelten Rezeptors;
(D) Messen der cytoplasmatischen Ca²⁺-Konzentration der Zellen; und
(E) Vergleichen der Ergebnisse aus den Messungen der Schritte (B) und (D),
wobei die Menge des Agonisten des G(alpha)q-gekoppelten Rezeptors, die eine untergrenzwertige Aktivität ergibt, diejenige ist, bei welcher das Verhältnis des Ergebnisses der Messung der cytoplasmatischen Ca²⁺-Konzentration der Zellen bei deren Behandlung mit einer Menge des Agonisten des G(alpha)q-gekoppelten Rezeptors zum Ergebnis der Messung der cytoplasmatischen Ca²⁺-Konzentration der Zellen bei deren Behandlung mit der gleichen Menge des Agonisten des G(alpha)q-gekoppelten Rezeptors und gleichzeitiger Behandlung der Zellen mit einer Menge eines Agonisten des G(alpha)i- und/oder G(alpha)o-gekoppelten Rezeptors, die zur vollständigen Aktivierung ausreicht, nicht größer als 1:3 ist.

4. In vitro Verfahren zur Identifizierung von Antagonisten eines G(alpha)i- und/oder G(alpha)o-gekoppelten Rezeptors, umfassend die Schritte:
(I) Bereitstellen von Zellen, die den G(alpha)i- und/oder G(alpha)o-gekoppelten Rezeptor und mindestens einen G(alpha)q-gekoppelten Rezeptor exprimieren; und
(II) Durchführen der Schritte (A) bis (E) gemäß dem Verfahren nach Anspruch 3, wobei im Schritt (C) als Antagonist des G(alpha)i- und/oder G(alpha)o-gekoppelten Rezeptors eine Testsubstanz, deren Aktivität bezüglich des G(alpha)i- und/oder G(alpha)o-gekoppelten Rezeptors getestet werden soll, verwendet wird.

5. Verfahren nach einem der Ansprüche 1 bis 4, wobei vor Schritt (a) bzw. vor Schritt (A) die Menge des Agonisten des G(alpha)q-gekoppelten Rezeptors, die eine untergrenzwertige Aktivität ergibt, durch die Schritte
(1) Behandeln der Zellen mit unterschiedlichen Mengen des Agonisten des G(alpha)q-gekoppelten Rezeptors;
(2) Messen der cytoplasmatischen Ca²⁺-Konzentration der Zellen für jede Menge des Agonisten von Schritt (1);
(3) Gleichzeitiges Behandeln der Zellen mit unterschiedlichen Mengen des Agonisten des G(alpha)q-gekoppelten Rezeptors und einer jeweils konstanten Menge des Agonisten des G(alpha)i- und/oder G(alpha)o-gekoppelten Rezeptors, die zur vollständigen Aktivierung des Rezeptors ausreicht;
(4) Messen der cytoplasmatischen Ca²⁺-Konzentration der Zellen für jede Menge des Agonisten des G(alpha)q-gekoppelten Rezeptors von Schritt (3); und
(5) Vergleichen der Ergebnisse der Messungen der Schritte (2) und (4) für jeweils gleiche Mengen des Agonisten des G(alpha)q-gekoppelten Rezeptors
ermittelt wird.

6. Verfahren nach Anspruch 5, wobei der Schritt (5) das Bilden des Verhältnisses aus den Ergebissen der Messungen der Schritte (2) und (4) umfasst.

7. Verfahren nach Anspruch 6, wobei die Menge des Agonisten des G(alpha)q-gekoppelten Rezeptors diejenige ist, bei welcher sich ein Verhältnis des Ergebnisses der Messung von Schritt (2) zum Ergebnis der Messung von Schritt (4) von nicht mehr als 1:3 ergibt.

8. Verfahren nach einem der Ansprüche 1 bis 7, wobei die Messung der cytoplasmatischen Ca²⁺-Konzentration mittels eines Ca²⁺-abhängigen Farbstoffes erfolgt.

9. Verfahren nach Anspruch 8, wobei der Farbstoff ein Fluoreszenzfarbstoff ist.

10. Verfahren nach Anspruch 9, wobei der Fluoreszenzfarbstoff aus der Gruppe, bestehend aus Molekülen der Familien Fura, Indo, Quin, Coumarinbenzothiazolen, Fluo, Calcium Green, Oregon Green, Calcium Orange, Calcium Crimson, Magnesium Green, Rhod und X-rhod, ausgewählt ist.

11. Verfahren nach einem der Ansprüche 1 bis 10, wobei der G(alpha)i- und/oder G(alpha)o-gekoppelte Rezeptor aus der Gruppe, bestehend aus Opioid-Rezeptoren, P2Y12, Rezeptoren der Edg-Familie, GABA-B-, Muscarin-M2-, -M4-, Dopamin-D2-, -D3-, -D4-, Histamin-H3-Rezeptoren, Rezeptoren der Serotonin-5HT1-Familie, C3a-, C5a-, fMLP-, CXCR1-5-, CCR1-9-, XCR1-, CX3CR1-, Neuropeptid-Y1-bis -Y6-, Somatostatin-Sst2-Rezeptoren, "Chemoattractant R-homologous molecule expressed on Th2 cells", Prostaglandin-E-Typ 3-, Adenosin-A1- und A-denosin-A3-Rezeptoren, ausgewählt ist.

12. Verfahren nach einem der Ansprüche 1 bis 11, wobei der G(alpha)q-gekoppelte Rezeptor aus der Gruppe, bestehend aus Muscarin-M1, -M3, -M5, Serotonin-5HT2, Bombesin, Cholecystokinin, Neurokinin, Prostaglandin-E-Typ I, Adenosin-A2B, P2Y1, P2Y2, P2Y4, P2Y6, P2Y11, Calcitonin, mGluR1-Rezeptoren, Angiotensin II Rezeptor 1 und "Protease Activated Receptor 1" ausgewählt ist.

13. Verfahren nach einem der Ansprüche 1 bis 12, wobei der G(alpha)i- und/oder G(alpha)o-gekoppelte Rezeptor und/oder der G(alpha)q-gekoppelte Rezeptor von den Zellen endogen exprimiert wird/werden.

14. Verfahren nach einem der Ansprüche 1 bis 13, wobei die Zellen mit einem Nukleinsäurekonstrukt zur Expression des G(alpha)i- und/oder G(alpha)o-gekoppelten Rezeptors und/oder des G(alpha)q-gekoppelten Rezeptors transfiziert sind.

15. Verfahren nach einem der Ansprüche 1 bis 14, wobei die Zellen aus stabilen Zellinien, primären Zellkulturen oder Gewebszellen ausgewählt sind.

16. Verfahren nach Anspruch 15, wobei die Zellinie aus der Gruppe, bestehend aus CHO, HEK293, THP-1, SH-SY5Y, Jurkat, HeLa, L-Zellen, A-10, Cos-7, NIH 3T3, ECV304, RBL, UMR 106, PC3, GH3, PC1 und IMR-32, ausgewählt ist.

17. Verfahren nach einem der Ansprüche 1 bis 16, wobei der Agonist des G(alpha)q-gekoppelten Rezeptors aus der Gruppe, bestehend aus Adenosin, AMP, ADP, ATP, Uridin, UMP, UDP, UTP, Cytosin, CMP, CDP, CTP, Guanosin, GMP, GDP, GTP, Thymidin, TMP, TDP, TTP, lonsin, IMP, IDP, ITP und Muscarin-Rezeptoragonisten, ausgewählt ist.

18. Verfahren nach einem der Ansprüche 1 oder 3 bis 17, wobei der Agonist des G(alpha)i- und/oder G(alpha)o-gekoppelten Rezeptors aus der Gruppe, bestehend aus GRT0777S, Damgo, Fentanyl, Serotonin, und Morphinen, ausgewählt ist.

19. Verfahren nach einem der Ansprüche 3 oder 5 bis 18, wobei der Antagonist des G(alpha)i- und/oder G(alpha)-gekoppelten Rezeptors Naloxon ist.

## Claims

1. An in vitro-method for measuring the activation of a G(alpha)i- and/or G(alpha)o-coupled receptor in cells which express at least one G(alpha)q-coupled receptor, comprising the steps:
a) simultaneously treating the cells with such an amount of an agonist of the G(alpha)q-coupled receptor, that an activity below the threshold value results, and with an agonist of the G(alpha)i- and/or G(alpha)o-coupled receptor; and
b) measuring the cytoplasmic Ca²⁺-concentration of the cells,
wherein the amount of the agonist of the G(alpha)q-coupled receptor which results in an activity below the threshold value is the amount at which the ratio of the result of the measurement of the cytoplasmic Ca²⁺-concentration of the cells in their treatment with an amount of the agonist of the G(alpha)q-coupled receptor to the result of the measurement of the cytoplasmic Ca²⁺-concentration of the cells in their treatment with the same amount of the agonist of the G(alpha)q-coupled receptor and simultaneous treatment of the cells with an amount of an agonist of the G(alpha)i- and/or G(alpha)o-coupled receptor which is sufficient for complete activation, is not greater than 1:3.

2. An in vitro-method for identifying agonists of a G(alpha)i- and/or G(alpha)o-coupled receptor, comprising the steps:
(i) providing cells which express the G(alpha)i- and/or G(alpha)o-coupled receptor and at least one G(alpha)q-coupled receptor; and
(ii) carrying out the steps (a) and (b) according to the method of claim 1, wherein in step (a) a test substance, whose activity with respect to the G(alpha)i- and/or G(alpha)o-coupled receptor is to be tested, is used as agonist of the G(alpha)i-and/or G(alpha)o-coupled receptor.

3. An in vitro-method for measuring the deactivation of a G(alpha)i- and/or G(alpha)o-coupled receptor in cells which express at least one G(alpha)q-coupled receptor, comprising the steps:
(A) simultaneously treating the cells with an amount of an agonist of the G(alpha)q-coupled receptor, such that an activity below the threshold value results, and with an amount of the agonist of the G(alpha)i- and/or G(alpha)o-coupled receptor which is just sufficient for complete activation; and
(B) measuring the cytoplasmic Ca²⁺-concentration of the cells;
(C) simultaneously treating the cells with the same amount of step (A) of the agonist of the G(alpha)q-coupled receptor, with the same amount of step (A) of the agonist of the G(alpha)i- and/or G(alpha)o-coupled receptor and with an antagonist of the G(alpha)i- and/or G(alpha)o-coupled receptor;
(D) measuring the cytoplasmic Ca²⁺-concentration of the cells;
(E) comparing the results of the measurements of the steps (B) and (D),
wherein the amount of the agonist of the G(alpha)q-coupled receptor which results in an amount below the threshold value is the amount at which the ratio of the result of the measurement of the cytoplasmic Ca²⁺-concentration of the cells in their treatment with an amount of the agonist of the G(alpha)q-coupled receptor to the result of the measurement of the cytoplasmic Ca²⁺-concentration of the cells in their treatment with the same amount of the agonist of the G(alpha)q-coupled receptor and simultaneous treatment of the cells with an amount of the G(alpha)i- and/or G(alpha)o-coupled receptor which is sufficient for complete activation, is not greater than 1:3.

4. An in vitro-method for identifying antagonists of a G(alpha)i- and/or G(alpha)o-coupled receptor, comprising the steps:
(I) providing cells which express the G(alpha)i- and/or G(alpha)o-coupled receptor and at least one G(alpha)q-coupled receptor; and
(II) carrying out the steps (A) to (E) according to the method of claim 3, wherein in step (C) a test substance, whose activity with respect to the G(alpha)i- and/or G(alpha)o-coupled receptor is to be tested, is used as antagonist of the G(alpha)i- and/or G(alpha)o-coupled receptor.

5. The method according the any one of claims 1 to 4, wherein prior to step (a) and prior to step (A), respectively, the amount of the agonist of the G(alpha)q-coupled receptor, which results in an activity below the threshold value, is determined by the steps:
(1) treating the cells with different amounts of the agonist of the G(alpha)q-coupled receptor;
(2) measuring the cytoplasmic Ca²⁺-concentration of the cells for each amount the agonist of step (1);
(3) simultaneously treating the cells with different amounts of the agonist of the G(alpha)q-coupled receptor and a respectively constant amount of the agonist of the G(alpha)i- and/or G(alpha)o-coupled receptor which is sufficient for complete activation;
(4) measuring the cytoplasmic Ca²⁺-concentration of the cells for each amount of the agonist of the G(alpha)q-coupled receptor of step (3); and
(5) comparing the results of the measurements of the steps (2) and (4) for respectively equal amounts of the agonist of the G(alpha)q-coupled receptor.

6. The method according to claim 5, wherein step (5) comprises calculation of the ratio of the results of the measurements of the steps (2) and (4).

7. The method according to claim 6, wherein the amount of the agonist of the G(alpha)q-coupled receptor is the amount at which a ratio of the result of the measurement of step (2) to the result of the measurement of step (4) of not greater than 1:3 results.

8. The method according to any one of claims 1 to 7, wherein the measurement of the cytoplasmic Ca²⁺-concentration is carried out using a Ca²⁺-dependent dye.

9. The method according to claim 8, wherein the dye is a fluorescent dye.

10. The method according to claim 9, wherein the fluorescent dye is selected from the group consisting of molecules of the families fura, indo, quin, coumarinbenzothiazoles, fluo, calcium green, oregon green, calcium orange, calcium crimson, magnesium green, rhod und X-rhod.

11. The method according to any one of claims 1 to 10, wherein the G(alpha)i- and/or G(alpha)o-coupled receptor is selected from the group consisting of opioid receptors, P2Y12, receptors of the Edg-family, GABA-B-, muscarine-M2-, -M4-, dopamine-D2-, -D3-, -D4-, histamine-H3-receptors, receptors of the serotonine-5HT1-family, C3a-, C5a-, fMLP-, CXCR1-5-, CCR1-9-, XCR1-, CX3CR1-, neuropeptide-Y1- to -Y6-, somatostatine-Sst2-receptors, "chemoattractant R-homologous molecule expressed on Th2 cells", prostaglandin-E-type 3-, adenosine-A1- and adenosine-A3-receptors.

12. The method according to any one of claims 1 to 11, wherein the G(alpha)q-coupled receptor is selected from the group consisting of muscarine-M1, -M3, -M5, serotonine-5HT2, bombesin, cholecystokinin, neurokinin, prostaglandin-E-type I, Adenosin-A2B, P2Y1, P2Y2, P2Y4, P2Y6, P2Y11, calcitonin, mGluRl-receptors, angiotensin II-receptor 1 and "Protease Activated Receptor 1 ".

13. The method according to any one of claims 1 to 12, wherein the G(alpha)i- and/or G(alpha)o-coupled receptor and/or the G(alpha)q-coupled receptor is/are endogenously expressed by the cells.

14. The method according to any one of claims 1 to 13, wherein the cells are transfected with a nucleic acid construct for the expression of the G(alpha)i- and/or G(alpha)o-coupled receptor and/or of the G(alpha)q-coupled receptor.

15. The method according to any one of claims 1 to 14, wherein the cells are selected from stable cell lines, primary cell cultures or tissue cells.

16. The method according to claim 15, wherein the cell line is selected from the group consisting of CHO, HEK293, THP-1, SH-SY5Y, Jurkat, HeLa, L-cells, A-10, Cos-7, NIH3T3, ECV304, RBL, UMR 106, PC3, GH3, PC1 and IMR-32.

17. The method according to any one of claims 1 to 16, wherein the agonist of the G(alpha)q-coupled receptor is selected from the group consisting of adenosine, AMP, ADP, ATP, uridine, UMP, UDP, UTP, cytosine, CMP, CDP, CTP, guanosine, GMP, GDP, GTP, thymidine, TMP, TDP, TTP, inosine, IMP, DP, ITP and muscarine-receptor-agonists.

18. The method according to any one of claims 1 or 3 to 17, wherein the agonist of the G(alpha)i- and/or G(alpha)o-coupled receptor is selected from the group consisting of GRT0777S, damgo, fentanyl, serotonin and morphines.

19. The method according to any one of claims 3 or 5 to 17, wherein the antagonist of the G(alpha)i- and/or G(alpha)o-coupled receptor is naloxone.

## Revendications

1. Méthode de mesure in vitro de l'activation d'un récepteur couplé à G(alpha)i- et/ou à G(alpha)o dans des cellules qui expriment au moins un récepteur couplé à G(alpha)q, comprenant les étapes qui consistent :
(a) à traiter simultanément les cellules avec une quantité d'un agoniste du récepteur couplé à G(alpha)q, calculée de manière qu'il en résulte une activité de valeur en-dessous de la limite, et avec un agoniste du récepteur couplé à G(alpha)i et/ou à G(alpha)o ; et
(b) à mesurer la concentration en Ca²⁺ dans le cytoplasme des cellules, la quantité de l'agoniste du récepteur couplé à G(alpha)q qui donne une activité de valeur en-dessous de la limite étant la quantité pour laquelle le rapport du résultat de la mesure de la concentration en Ca²⁺ dans le cytoplasme des cellules lors de leur traitement avec une quantité de l'agoniste du récepteur couplé à G(alpha)q au résultat de la mesure de la concentration en Ca²⁺ dans le cytoplasme des cellules lors de leur traitement avec la même quantité de l'agoniste du récepteur couplé à G(alpha)q et du traitement simultané des cellules avec une quantité d'un agoniste du récepteur couplé à G(alpha)i et/ou à G(alpha)o qui suffit pour l'activation totale, n'est pas supérieur à 1:3.

2. Méthode d'identification in vitro d'agonistes d'un récepteur couplé à G(alpha)i et/ou à G(alpha)o, qui comprend les étapes consistant .
(i) à rendre disponibles des cellules qui expriment le récepteur couplé à G(alpha)i et/ou à G(alpha)o et au moins un récepteur couplé à G(alpha)q ; et
(ii) à conduire les étapes (a) et (b) conformément à la méthode selon la revendication 1, en utilisant dans l'étape (a) comme agoniste du récepteur couplé à G(alpha)i et/ou à G(alpha)o, une substance d'essai dont l'activité vis-à-vis du récepteur couplé à G(alpha)i et/ou à G(alpha)o doit être évaluée.

3. Méthode de mesure in vivo de la désactivation d'un récepteur couplé à G(alpha)i et/ou à G(alpha)o dans des cellules qui expriment au moins un récepteur couplé à G(alpha)q, comprenant les étapes qui consistent .
(A) à traiter simultanément les cellules avec une quantité d'un agoniste du récepteur couplé à G(alpha) q, calculée de manière qu'il en résulte une activité de valeur en-dessous de la limite, et avec une quantité d'un agoniste du récepteur couplé à G (alpha) i et/ou à G (alpha) o exactement suffisante pour l'activation totale ;
(B) à mesurer la concentration en Ca²⁺ dans le cytoplasme des cellules ;
(C) à traiter simultanément les cellules avec la même quantité de l'agoniste du récepteur couplé à G(alpha)q de l'étape (A) avec la même quantité de l'agoniste du récepteur couplé à G(alpha)i et/ou à G(alpha)o de l'étape (A) et avec un antagoniste du récepteur couplé à G(alpha)i et/ou à G(alpha)o ;
(D) à mesurer la concentration en Ca²⁺ dans le cytoplasme des cellules ; et
(E) à comparer les résultats des mesures des étapes (B) et (D),
la quantité de l'agoniste du récepteur couplé à G(alpha)q qui donne une activité de valeur en-dessous de la limite étant la quantité pour laquelle le rapport du résultat de la mesure de la concentration en Ca²⁺ dans le cytoplasme des cellules lors de leur traitement avec une quantité de l'agoniste du récepteur couplé à G(alpha)q au résultat de la mesure de la concentration en Ca²⁺ dans le cytoplasme des cellules lors de leur traitement avec la même quantité de l'agoniste du récepteur couplé à G(alpha)q et du traitement simultané des cellules avec une quantité d'un agoniste du récepteur couplé à G(alpha)i et/ou à G(alpha)o qui suffit pour l'activation totale, n'est pas supérieur à 1:3.

4. Méthode d'identification in vitro d'antagonistes d'un récepteur couplé à G(alpha)i et/ou à G(alpha)o, comprenant les étapes qui consistent .
(i) à rendre disponibles des cellules qui expriment le récepteur couplé à G(alpha)i et/ou à G(alpha)o et au moins un récepteur couplé à G(alpha)q ; et
(ii) à conduire les étapes (A) à (E) conformément à la méthode selon la revendication 3, en utilisant dans l'étape (C) comme antagoniste du récepteur couplé à G(alpha)i et/ou à G(alpha)o, une substance d'essai dont l'activité par rapport au récepteur couplé à G(alpha)i et/ou à G(alpha)o doit être évaluée.

5. Méthode suivant l'une des revendications 1 à 4, dans laquelle, préalablement à l'étape (a) ou respectivement à l'étape (A), la quantité de l'agoniste du récepteur couplé à G(alpha)q, qui donne une activité de valeur en-dessous de la limite, est déterminée par les étapes consistant:
(1) à traiter les cellules avec différentes quantités de l'agoniste du récepteur couplé à G(alpha)q ;
(2) à mesurer la concentration en Ca²⁺ dans le cytoplasme des cellules pour chaque quantité d'agoniste de l'étape (1) ;
(3) à traiter simultanément les cellules avec différentes quantités de l'agoniste du récepteur couplé à G(alpha)q et une quantité chaque fois constante de l'agoniste du récepteur couplé à G(alpha)i et/ou à G(alpha)o, qui est suffisante pour l'activation totale du récepteur ;
(4) à mesurer la concentration en Ca²⁺ dans le cytoplasme des cellules pour chaque quantité d'agoniste du récepteur couplé à G(alpha)q de l'étape (3) ; et
(5) à comparer les résultats des mesures des étapes (2) et (4) pour, à chaque fois, les mêmes quantités de l'agoniste du récepteur couplé à G(alpha)q.

6. Méthode suivant la revendication 5, dans laquelle l'étape (5) comprend la formation du rapport des résultats des mesures des étapes (2) et (4).

7. Méthode suivant la revendication 6, dans laquelle la quantité de l'agoniste du récepteur couplé à G(alpha)q est celle pour laquelle il s'établit un rapport du résultat de la mesure de l'étape (2) au résultat de la mesure de l'étape (4) ne dépassant pas 1:3.

8. Méthode suivant l'une des revendications 1 à 7, dans laquelle la mesure de la concentration en Ca²⁺ dans le cytoplasme est effectuée au moyen d'un colorant dépendant de Ca²⁺.

9. Méthode suivant la revendication 8, dans laquelle le colorant est un colorant fluorescent.

10. Méthode suivant la revendication 9, dans laquelle le colorant fluorescent est choisi dans le groupe constitué de molécules des familles fura, indo, quin, coumarinbenzothiazoles, fluo, calcium green, oregon green, calcium orange, calcium crimson, magnesium green, rhod et X-rhod.

11. Méthode suivant l'une des revendications 1 à 10, dans laquelle le récepteur couplé à G(alpha)i et/ou à G(alpha)o est choisi dans le groupe constitué des récepteurs opiacés, P2Y12, des récepteurs de la famille Edg, des récepteurs du GABA-B, des récepteurs de la muscarine M2-, M4-, des récepteurs dopaminergiques D2-, D3, D4, des récepteurs histaminiques H3, des récepteurs de la famille de la sérotonine-SHT1, des récepteurs C3a, C5a, fMLP, CXCR1-5, CCR1-9, XCR1, CX3CR1, des récepteurs de neuropeptides Y1 à Y6, des récepteurs de somatostatine-Sst2, des "chemoattractant R-homologous molecule expressed on Th2 cells", des récepteurs de prostaglandine E-type 3, des récepteurs d'adénosine A1 et d'adénosine A3.

12. Méthode suivant l'une des revendications 1 à 11, dans laquelle le récepteur couplé à G(alpha)q est choisi dans le groupe comprenant le récepteur de muscarine M1, muscarine M3, muscarine M5, sérotonine-SHT2, bombésine, cholécystokinine, neurokinine, prostaglandine E-type 1, adénosine A2B, P2Y1, P2Y2, P2Y4, P2Y6, P2Y11, calcitonine, les récepteurs de mGluR1, le récepteur d'angiotensine II et le "protease activated receptor 1".

13. Méthode suivant l'une des revendications 1 à 12, dans laquelle le récepteur couplé à G(alpha)i et/ou à G(alpha)o et/ou le récepteur couplé à G(alpha)q est ou sont exprimés de façon endogène par les cellules.

14. Méthode suivant l'une des revendications 1 à 13, dans laquelle les cellules sont transfectées avec un acide nucléique d'assemblage pour l'expression du récepteur couplé à G(alpha)i et/ou à G(alpha)o et/ou du récepteur couplé à G(alpha)q.

15. Méthode suivant l'une des revendications 1 à 14, dans laquelle les cellules sont choisies entre des lignées cellulaires stables, des cultures de cellules primaires et des cellules de tissu.

16. Méthode suivant la revendication 15, dans laquelle la lignée cellulaire est choisie dans le groupe constitué de CHO, HEK293, THP-1, SH-SY5Y, Jurkat, HeLa, cellules L, A-10, Cos-7, NIH 3T3, ECV304, RBL, UMR 106, PC3, GH3, PC1 et IMR-32.

17. Méthode suivant l'une des revendications 1 à 16, dans laquelle l'agoniste du récepteur couplé à G(alpha)q est choisi dans le groupe constitué d'adénosine, AMP, ADP, ATP, uridine, UMP, UDP, UTP, cytosine, CMP, CDP, CTP, guanosine, GMP, GDP, GTP, thymidine, TMP, TDP, TTP, lonsine, IMP, IDP, ITP et agonistes des récepteurs muscariniques.

18. Méthode suivant l'une des revendications 1 ou 3 à 17, dans laquelle l'agoniste du récepteur couplé à G(alpha)i et/ou à G(alpha)o est choisi dans le groupe constitué de GRT0777S, damgo, fentanyl, sérotonine et morphine.

19. Méthode suivant l'une des revendications 3 ou 5 à 18, dans laquelle l'agoniste du récepteur couplé à G(alpha)i et/ou à G(alpha)o est la naloxone.
